# EUROPEAN PATENT APPLICATION

(11) **EP 2 280 085 A2**
(43) Date of publication of application: **02.02.2011**
(21) Application number: 10180021.7
(22) Date of filing: 01.11.2005
(51) Int. Cl.: C12Q 1/68

(54) **Compositions and methods for diagnosing colon disorders**

(30) Priority: 01.11.2004 US 623771 P; 26.01.2005 US 646592 P
(62) Divisional of application: 05824553.1
(71) Applicant: George Mason University, Fairfax, VA 22030 (US)
(72) Inventor: Gillevet, Patric Georg Mason University, Manassas, VA 20110 (US)
(74) Representative: Vossius & Partner

(57) **Abstract**

The present invention relates to methods and compositions for diagnosing, monitoring, prognosticating, analyzing, etc., polymicrobial diseases. The present invention also relates to the microbial community present in the digestive tract and lumen in normal subjects, and subjects with digestive tract diseases, especially diseases of the colon, such as inflammatory bowel disease, including ulcerative colitis, Crohn's syndrome, and pouchitis. The present invention especially relates to compositions and methods for diagnosing and prognosticating the mentioned diseases and conditions, e.g., to determine the presence of the disease in a subject, to determine a therapeutic regimen, to determine the onset of active disease, to determine the predisposition to the disease, etc.

## Description

### BACKGROUND OF THE INVENTION

Ulcerative Colitis and Crohn's disease are chronic inflammatory diseases of the colon and rectum. Although corticosteroids, aminosalicylates, and immunomodulators have provided some benefit in treatment of ulcerative colitis, restorative proctocolectomy ileal-pouch anal anastamosis (RP/IPAA) remains the gold standard for management of chronically active and steroid-refractory disease. The most common and debilitating complication of IPAA is symptomatic inflammation of the ileal reservoir, or pouchitis. Prior studies have demonstrated a significant decrease in quality of life (IBDQ and SF-36) when RP/IPAA is complicated by pouchitis. The incidence of pouchitis is between 30-50% up to 5 years postoperatively, with the majority of initial cases in the first 3-6 months. Clinically, pouchitis is characterized by increased stool frequency, fecal urgency, rectal bleeding, and malaise. However, the diagnosis of pouchitis is a combination of specific clinical, endoscopic, and histologic criteria. There is much debate as to whether pouchitis is an extension of the Ulcerative Colitis or a distinct disease entity. There has been no data to strongly favor either. Although many theories have been proposed, the precise mechanism of disease in pouchitis remains elusive. The dramatic clinical response to antibiotics in pouchitis suggests that microflora may play a causal role. Despite an 80% initial response to antibiotics, 60% of patients have recurring episodes of pouchitis and up to 30% of patients develop chronic symptomatic pouchitis. There have been no studies to date identifying any specific microfloral pattern or organism in the pathogenesis of pouchitis. In this study, we introduce Amplicon Length Heterogeneity, a novel culture-independent technique for detailed microfloral characterization in pouchitis.

### DESCRIPTION OF THE DRAWING

Fig. 1. Histogram of Crohns tissue and lumen ALH Fingerprints.
Fig. 2. Histogram of Ulcerative Colitis tissue and lumen ALH Fingerprints.
Fig. 3. Principal coordinate analysis (PCO) of Crohns and Ulcerative Colitis ALH Fingerprints.
Fig. 4. Histogram of normal Pouch and Pouchitis tissue ALH Fingerprints.
Fig. 5. Principle coordinate analysis (PCO) af Pouchitis ALH Fingerprints.
Fig. 6. Identification of peaks in normal Pouch and Pouchitis Histogram.

### DESCRIPTION OF THE INVENTION

The present invention relates to methods and compositions for diagnosing, monitoring, prognosticating, analyzing, etc., polymicrobial diseases. A polymicrobial disease is a disease or condition that is associated with the presence of at least two different microbes, including, e.g., associations between bacteria-bacteria, virus-virus, parasite-parasite, bacteria-virus, bacteria-parasite, and virus-parasite. A preferred method of determining the microbial community present in a polymicrobial disease is amplicon length heterogeneity ("ALH").

Examples of polymicrobial diseases include, but are not limited to, e.g., co-infection of Borrelia and Ehrlichia in Lyme borreliosis; mixed viral-bacterial infections during influenza pandemics; respiratory diseases; gastroenteritis; conjunctivitis; keratitis; hepatitis; periodontal diseases; genital infections; intra-abdominal infections; inflammatory bowel diseases; urinary tract infections; necrotizing soft-tissue infection.

The present invention also relates to the microbial community present in the digestive tract and lumen in normal subjects, and subjects with digestive tract diseases, especially diseases of the colon, such as inflammatory bowel disease, including ulcerative colitis, Crohn's syndrome, and pouchitis. The present invention especially relates to compositions and methods for diagnosing, prognosticating, and/or monitoring the disease progression of the mentioned diseases and conditions, e.g., to determined the presence of the disease in a subject, to determine a therapeutic regimen, to determine the onset of active disease, to determine the predisposition to the disease, to determine the course of the disease, etc.

The present invention provides methods for diagnosing and monitoring the disease progression of inflammatory bowel diseases, such as ulcerative colitis, Crohn's disease, or pouchitis, comprising determining the presence or absence of microbes, such as bacteria, in a colon or lumen sample obtained from said subject. The invention is not limited to how the determination is carried out; any suitable method can be used. The term "microbe" includes viruses, bacteria, fungi, and protists. Although the disclosure below may be written in terms of bacteria, any microbe can be used.

The present invention relates to any composition or method which is suitable for detecting a microbial community in a sample (e.g., from a subject having a polymicrobial disease), such as a digestive tract, lumen, or stool sample. A lumen sample is from interior of the intestine.

Any marker which is suitable for identifying and distinguishing a microbial type can be utilized in accordance with present invention. These methods can involve detection of nucleic acid (e.g., DNA, RNA, mRNA, tRNA, rRNA, etc), protein (e.g., using antibodies, protein binding reagents), and any other bio-molecule (e.g., lipid, carbohydrates, etc) that is useful for specifically determining the presence or absence of bacteria in a sample. Any variable indicator or non-coding segment (e.g., repetitive elements, etc.) can also be used, as well as indicator genes. ITS regions can be utilized in fungi.

Standard culture methods can also be utilized, where bacteria and other microorganisms are identified by culturing them on a media, e.g., using a selective media (e.g., comprising a bacteria-specific carbon source) and/or where microorganisms are identified by their growth characteristics, morphology, and other criteria typically used to determine cell identity and phylogenetic classification. Any of these methods can also be used in combination with cytological and histological methods, where biopsy samples or cultured samples can be stained and visualized (e.g., by sectioning, or by mounting on a slide or other carrier).

As mentioned, the compositions and methods are useful for diagnostic and prognostic purposes associated with polymicrobial diseases, such as inflammatory bowel diseases, including ulcerative colitis, Crohn's disease, and pouchitis. The markers and fingerprints can be utilized to diagnose the diseases, and distinguish them from other diseases of the digestive tract. They can also be used for assessing disease status, severity, and prognosis, alone, or in combination with other tests. For example, the markers can be used in conjunction with the Crohn's disease activity index (CDAI) or the criteria of Trulove and Witts for assessing disease activity in ulcerative colitis. The information about microorganismal status can also be used to determine when to initiate drug treatment or other therapeutic regimens.

The methods and compositions can also be used to monitor the course of the disease in a subject under treatment or monitor the progression of the disease, irrespective of the treatment regimen. For example, patients with inflammatory bowel syndromes may show spontaneous or drug-induced remissions. To monitor the course of the remission and determine when the disease is active, samples can be obtained periodically, and assayed to determine the appearance of the particular microbial markers or fingerprints in the intestinal tissue, lumen, colonic wash, mucosal samples, or stool.

Assessment of the microbial community can be performed on any sample obtained from a subject, including from lumen, colonic wash, intestinal tissue, intestinal mucosa, gastric tissue, gastric mucosa, stool, etc. Samples can be obtained from any part of the digestive tract, especially the small and large intestines. The large intestine or colon is the part of the intestine from the cecum to the rectum. It is divided into eight sections: the cecum, the appendix, the ascending colon, the transverse colon, the descending colon, the sigmoid colon, the rectum, and the anus. A colonic wash is the fluid left in the intestine after a subject has been given a laxative. The intestinal mucosa is the surface lining of the intestinal tract. Subjects include, e.g., animals, humans, non-human primates, mammals, monkeys, livestock, sheep, goats, pigs, pets (e.g., dogs, cats), small animals, reptiles, birds, etc.

Any suitable method can be utilized to obtain samples from the intestine. Endoscopic biopsy is common method in which a fiber optic endoscope is inserted into the gastrointestinal tract through a natural body orifice. The lining of the intestine is directly visualized and a sample is pinched off with forceps attached to a long cable that runs inside the endoscope. Suitable endoscopes and instruments for removing biopsy samples are well known, and include those disclosed in, e.g., U.S. Pat. Nos. 6,632,182, and 6,443,909.

Table 3 summarizes bacteria which have been detected in mucosa tissue and lumen from control subjects, and subjects having Crohn's disease or ulcerative. Table 5 summarizes bacteria which have been detected in the mucosa and lumen of subjects having pouchitis and pouchitis control (subjects with restorative proctocolectomy, but without post-operative complications). PCR amplicons were cloned and sequenced from these samples. Briefly, DNA was extracted from each pooled sample. The pooled DNA from mucosa comprised DNA from mucosal and other gastrointestinal cells, as well as the bacteria. The first two variable regions of the 16S ribosomal RNA were amplified using universal Eubacterial primers. Subsequently, the amplification mixture was separated and characterized on a fingerprinting gel. The resulting picture of the gel or tabular compilation of the data (see, e.g., Tables 1, 2, and 4) - comprising discrete, individual bands (PCR amplicons) - can be referred to as the "ALH fingerprint." The ALH fingerprint can be further characterized by identifying the length of the individual replicons that comprise it and/or their specific nucleotides sequences. Amplicons from the microbial community can then be cloned and sequenced, where the sequence is correlated with a particular bacterial group, species, or strain. Using this method, the abundance of the clones from each species is proportional to their abundance in the corresponding community, and can be correlated to peaks in the ALH fingerprint. The sequence data can be used to search the Ribosomal database (RDP) using a standard sequence search tool (Megablast) available from the National Center for Biotechnology Information (NCBI) at NIH. See, e.g., Cole JR, Chai B, Marsh TL, Farris RJ, Wang Q, Kulam SA, Chandra S, McGarrell DM, Schmidt TM, Garrity GM, Tiedje JM. The Ribosomal Database Project (RDP-II): previewing a new autoaligner that allows regular updates and the new prokaryotic taxonomy. Nucleic Acids Res 2003 Jan 1;31(1):442-3. The RDP number obtained from the search results can be parsed using a custom PERL script to classify the Division, Subdivision, Group, and Subgroup of each clone, and the results can be tabulated, and imported into EXCEL or other suitable databases.

### Nucleic acid detection methods

Detection methods have a variety of applications, including for diagnostic, prognostic, forensic, and research applications. To accomplish nucleic acid detection, a polynucleotide in accordance with the present invention can be used as a "probe." The term "probe" or "polynucleotide probe" has its customary meaning in the art, e.g., a polynucleotide which is effective to identify (e.g., by hybridization), when used in an appropriate process, the presence of a target polynucleotide to which it is designed. Identification can involve simply determining presence or absence, or it can be quantitative, e.g., in assessing amounts of a polynucleotide (e.g., copies of a ribosomal RNA) present in a sample. As explained in more detail below, any suitable method can be used, including, but limited to, ALH, PCR, nucleotide sequencing, Southern blot, and or DNA microarrays (e.g., where a microarray comprises a plurality of sequences specific for one or more bacteria of the present invention).

Assays can be utilized which permit quantification and/or presence/absence detection of a target nucleic acid in a sample. Assays can be performed at the single-cell level, or in a sample comprising many cells, where the assay is "averaging" expression over the entire collection of cells and tissue present in the sample. Any suitable assay format can be used, including, but not limited to, e.g., Southern blot analysis, Northern blot analysis, polymerase chain reaction ("PCR") (e.g., Saiki et al., Science, 241:53, 1988; U.S. Pat. Nos. 4,683,195, 4,683,202, and 6,040,166; PCR Protocols: A Guide to Methods and Applications, Innis et al., eds., Academic Press, New York, 1990), reverse transcriptase polymerase chain reaction ("RT-PCR"), anchored PCR, rapid amplification of cDNA ends ("RACE") (e.g., Schaefer in Gene Cloning and Analysis: Current Innovations, Pages 99-115, 1997), ligase chain reaction ("LCR") (EP 320 308), one-sided PCR (Ohara et al., Proc. Natl. Acad. Sci., 86:5673-5677, 1989), indexing methods (e.g., U.S. Pat. No. 5,508,169), *in situ* hybridization, differential display (e.g., Liang et al., Nucl. Acid. Res., 21:3269-3275, 1993; U.S. Pat. Nos. 5,252,311, 5,599,672 and 5,965,409; WO97/18454; Prashar and Weissman, Proc. Natl. Acad. Sci., 93:659-663, and U.S. Pat. Nos. 6,010,850 and 5,712,126; Welsh et al., Nucleic Acid Res., 20:4965-4970, 1992, and U.S. Pat. No. 5,487,985) and other RNA fingerprinting techniques, nucleic acid sequence based amplification ("NASBA") and other transcription based amplification systems (e.g., U.S. Pat. Nos. 5,409,818 and 5,554,527; WO 88/10315), polynucleotide arrays (e.g., U.S. Pat. Nos. 5,143,854, 5,424,186; 5,700,637, 5,874,219, and 6,054,270; PCT WO 92/10092; PCT WO 90/15070), Qbeta Replicase (PCT/US87/00880), Strand Displacement Amplification ("SDA"), Repair Chain Reaction ("RCR"), nuclease protection assays, subtraction-based methods, Rapid-Scan™, etc. Additional useful methods include, but are not limited to, e.g., template-based amplification methods, competitive PCR (e.g., U.S. Pat. No. 5,747,251), redox-based assays (e.g., U.S. Pat. No. 5,871,918), Taqman-based assays (e.g., Holland et al., Proc. Natl. Acad, Sci., 88:7276-7280, 1991; U.S. Pat. Nos. 5,210,015 and 5,994,063), real-time fluorescence-based monitoring (e.g., U.S. Pat. 5,928,907), molecular energy transfer labels (e.g., U.S. Pat. Nos. 5,348,853, 5,532,129, 5,565,322, 6,030,787, and 6,117,635; Tyagi and Kramer, Mature Biatech., 14:303-309, 1996). Any method suitable for single cell analysis of polynucleotide or protein expression can be used, including *in situ* hybridization, immunocytochemistry, MACS, FACS, flow cytometry, etc. For single cell assays, expression products can be measured using antibodies, PCR, or other types of nucleic acid amplification (e.g., Brady et al., Methods Mol. & Cell. Biol. 2, 17-25, 1990; Eberwine et al., 1992, Proc. Natl. Acad. Sci., 89, 3010-3014, 1992; U.S. Pat. No. 5,723,290). These and other methods can be carried out conventionally, e.g., as described in the mentioned publications.

Many of such methods may require that the polynucleotide is labeled, or comprises a particular nucleotide type useful for detection. The present invention includes such modified polynucleotides that are necessary to carry out such methods. Thus, polynucleotides can be DNA, RNA, DNA:RNA hybrids, PNA, etc., and can comprise any modification or substituent which is effective to achieve detection.

The present invention provides methods for diagnosing or prognosticating ulcerative colitis, Crohn's disease, or pouchitis, or in a subject, comprising, one or more of the following steps in any effective order, e.g., contacting a gastrointestinal tissue or lumen sample comprising nucleic acid with a polynucleotide probe which is specific for at least one bacteria under conditions effective for said probe to hybridize specifically with said nucleic acid, and detecting hybridization between said probe and said nucleic acid, wherein the presence of one or more bacteria selected from the following group said bacteria indicates the disease presence or the disease status of ulcerative colitis, Crohn's disease, or Pouchitis. The method can further comprise obtaining a colon sample, e.g., by endoscopic biopsy, and/or extracting the nucleic acid from the sample.

The phrases "specific for" or "specific to" a microbe has a functional meaning that indicates the probe (or antibody if it used in a protein context) can be used to identify the presence of the target microbe in a sample and distinguish it from non-target microbe. It is also specific in the sense that it can be used to detect target microbe above background noise ("non-specific binding"). This same definition is also applicable to a polynucleotide or antibody probe. Probes can also be described as being specific for a sequence, where a specific sequence is a defined order of nucleotides (or amino acid sequences, if it is a polypeptide sequence) that occurs in the polynucleotide.

The phrase "hybridize specifically" indicates that the hybridization between single-stranded polynucleotides is based on nucleotide sequence complementarity. The effective conditions are selected such that the probe hybridizes to a pre-selected and/or definite target nucleic acid in the sample. For instance, if detection of a polynucleotide for a ribosomal RNA is desired, a probe can be selected which can hybridize to the target ribosomal RNA under high stringent conditions, without significant hybridization to other non-target sequences in the sample. For example, the conditions can be selected routinely which require 100% or complete complementarity between the target and probe.

Contacting the sample with probe can be carried out by any effective means in any effective environment. It can be accomplished in a solid, liquid, frozen, gaseous, amorphous, solidified, coagulated, colloid, etc., mixtures thereof, matrix. For instance, a probe in an aqueous medium can be contacted with a sample which is also in an aqueous medium, or which is affixed to a solid matrix, or vice-versa.

Generally, as used throughout the specification, the term "effective conditions" means, e.g., the particular milieu in which the desired effect is achieved, such as hybridization between a probe and its target, or antibody binding to a target protein. Such a milieu, includes, e.g., appropriate buffers, oxidizing agents, reducing agents, pH, co-factors, temperature, ion concentrations, suitable age and/or stage of cell (such as, in particular part of the cell cycle, or at a particular stage where particular genes are being expressed) where cells are being used, culture conditions (including substrate, oxygen, carbon dioxide, etc.). When hybridization is the chosen means of achieving detection, the probe and sample can be combined such that the resulting conditions are functional for said probe to hybridize specifically to nucleic acid in said sample.

For detecting the presence of a probe specifically hybridized to a target, any suitable method can be used. For example, polynucleotides can be labeled using radioactive tracers such as ³²P, ³⁵S, ³H, or ¹⁴C, to mention some commonly used tracers. Non-radioactive labeling can also be used, e.g., biotin, avidin, digoxigenin, antigens, enzymes, or substances having detectable physical properties, such as fluorescence or the emission or absorption of light at a desired wavelength, etc.

Any test sample in which it is desired to identify the presence or absence of bacteria can be used, including, e.g., blood, urine, saliva, lumen (for extracting nucleic acid, see, e.g., U.S. Pat. No. 6,177,251), stool, swabs comprising tissue, biopsied tissue, tissue sections, cultured cells, intestinal wash, colonic wash, intestinal mucosa, etc

The results for any of the assays mentioned herein (including the assays in other sections below) can be with respect to a control sample. For example, an increase or decrease can be with respect (in comparison) to a normal lumen or mucosa sample. The normal sample can be from the same patient, but from an unafflicted region or period (e.g., when the patient is in remission). It can also be from a standard value that is calculated based on a normalized population of individuals. Standard statistics can be utilized to determine whether the values are significant.

The present invention also provides methods for nucleic acid fingerprinting the community of microbes present in a sample, e.g., using universal primers to the microorganisms in question, whether they be Eubacteria, Archaebacteria, Fungi, or Protists. Since each sample contains a distinctive population of microbes that is representative of the disease, sampling the nucleic acids from the microbes can produce a distinctive array of polynucleotide fragments associated with the disease. These can be presented by any physical characteristic, including size, sequence, mobility, molecular weight (e.g., using mass spectroscopy), etc. Any fingerprinting method can be used, including, e.g., AFLP, ALH, LH-PCR, ARISA, RAPD, etc. Tables 1, 2, and 4 show the frequency of amplicons in various control and disease samples. Although one particular amplicons may not be diagnostic of the condition 100% of the time, using multiple amplicons increases the diagnostic certainty. Moreover, when a condition is being monitored, it may be advantageous to monitor a complex fingerprint (such as shown in Table 1) a it differs from one sampling time to another.

Along these lines, the present invention provides method for diagnosing, prognosticating, or monitoring the disease progression of a polymicrobial disease (e.g., an inflammatory bowel disease, such as ulcerative colitis, pouchitis, or Crohn's disease), comprising one or more of the following steps in any effective order, e.g., performing an amplification reaction on a sample comprising nucleic acid with at least two polynucleotide probe primers which are effective for amplifying the microbial community present in said sample, and detecting the reaction products of said amplification reaction, whereby said reaction products comprise a pattern that indicate the presence of the disease or the disease status.

By "disease status," it is meant the relative condition of the disease as compared to its condition at a previous time. For example, when sample reaction products differ (e.g., in quantity or size) from a period of disease severity, this would indicate that the disease status of the subject had changed. The reaction products may show a difference before the subject actually manifests symptoms of the disease, and therefore can be used prognostically to predict a relapse. Similarly, a change in the reaction products can also indicate that the disease is improving and/or responding to a treatment regime.

The term "amplification" indicates that the nucleic acid sequences are increased in copy number to an amount or quantity at which they can be detected. Amplification can be carried out conventionally, using any suitable technique, including polymerase chain reaction (PCR), NASBA (e.g., using T7 RNA polymerase), LCR (ligation chain reaction), LH-PCR, ARISA.

Total nucleic can be extracted from a sample, or the sample can be treated in such a way to preferentially extract nucleic acid only from the microbes that are present in it.
DNA extractions can be performed with commercially available kits, such as the Bia101 kit from Qbiogene, Inc, Montreal, Quebec. To prevent contamination by multiple samples during the homogenization process of a sample, each individual sample can be processed separately and completely leading to high yield DNA extractions.

In certain embodiments of the present invention, ribosomal RNA ("rRNA") can be used to distinguish and detect bacteria. For example, bacterial ribosomes are comprised of a small and large subunit, each which is further comprised of ribosomal RNAs and proteins. The rRNA from the small subunit can be referred to as SSU rRNA, and from the larger subunit as LSU rRNA. A large number of rRNAs have been sequenced, and these are publicly available in various accessible databases. See, e.g., Wuyts et al., The European database on small subunit ribosomal RNA, Nucleic Acids Res., 30, 183-185, 2002; Cole et al., The Ribosomal Database Project (RDP-II): previewing a new autoaligner that allows regular updates and the new prokaryotic taxonomy. Nucleic Acids Res., 31(1): 442-3,2003. See, also, http://rdp.cme.msu.edu/html/.accessed on June 14, 2004, Any rRNA can be used as a marker, including, but not limited to, 16S, 23S, and 5S.

Primer sequences to rRNA can be designed routinely to detect specific species of bacteria, or to detect groups of bacteria, e.g., where a conserved sequence is characteristic of a bacterial group. ALH-PCR can be accomplished routinely, e.g., using a fluorescently labeled forward primer 27F (5'-[6FAM] AGAGTTTGATCCTGGCTCAG-3') (SEQ ID NO:37) and unlabeled reverse primer 338R' (5'-GCTGCCTCCCGTAGGAGT-3') (SEQ ID NO:38). Both primers are highly specific for Eubacteria (Lane, D.J., 16S/23S rRNA Sequencing, in Nucleic Acid Techniques in Bacterial Systematics, E.S.a.M. Goodfellow, Editor. 1991, John Wiley & Sons Ltd: West Sussex, England. p. 115-175).

Primers can also be utilized which amplify the corresponding region in Archae (Burggraf, S., T. Mayer, R. Amann, S. Schadhauser, C.R. Woese and K.O. Stetter, Identifying Members of the Domain Archaea with rRNA-Targeted Oligonicleotide Probes. App. Environ. Microbiol., 1994. 60: p. 3112-3119), Eukaryotes (Rowan, R. and D.A. Powers, Ribosomal RNA sequences and the diversity of symbiotic dinoflagellates (zooxanthellae). Proceedings of the National Academy of Sciences, USA, 1992. 89: p. 3639-3643), and Fungi (Borneman, J. and J. Hartin, PCR primers that amplify fungal rRNA genes from Environmental Samples. App. Environ. Microbiol., 2000. 66(10): p. 4356-4360).

Primers can be selected from any nucleic acid of the infectious agent, including from rRNA, tRNA, genomic DNA, etc. The primers can be to variable regions, helices, conserved regions, etc.

Selected primers can be utilized in amplicon length heterogeneity ("ALH") to generate fingerprints that characterize the bacterial community (Ritchie, N.J., et al., Use of Length Heterogeneity PCR and Fatty Acid Methyl Ester Profiles to Characterize Microbial Communities in Soil. Applied and Environmental Microbiology, 2000. 66(4): p. 1668-1675; Suzuki, M., M.S. Rappe, and S.J. Giovannoni, Kinetic bias in estimates of coastal picoplankton community structure obtained by measurements of small-subunit rRNA gene PCR amplicon length heterogeneity. Applied and Environmental Microbiology [Appl. Environ. Microbiol.]. 1998. 64(11): p. 4522-4529; Litchfield, C.D. and P.M. Gillevet, Microbial diversity and complexity in hypersaline environments: A preliminary assessment, Journal of Industrial Microbiology & Biotechnology [J. Ind. Microbiol. Biotechnol.]. 2002. 28(1): p. 48-55; Mills, D.K., et al., A Comparison of DNA Profiling Techniques for Monitoring Nutrient Impact on Microbial Community Composition during Bioremediation of Petroleum Contaminated Soils. J. Microbiol. Method, 2003. 54: p. 57-74).

Individual primers can be utilized or a mixture, e.g., comprising degenerate sequences, sequences from one or more group, multiplex reaction where different groups are assessed using primers labeled with different fluorescent tags etc.

The reaction products (i.e., the fragments which are detected after the amplification reaction) can be analyzed by statistical analysis, such as PCO analysis (see Examples) to determine which products are diagnostic of the disease.

### Polypeptide detection

The present invention also provides compositions and methods for detecting polypeptides and other biomolecules that are characteristic of the microbial population. For example, the present invention provides methods for diagnosing or prognosticating ulcerative colitis, pouchitis, or Crohn's disease comprising:, one or more of the following steps in any effective order, e.g., contacting a sample comprising protein with an antibody which is specific for a bacteria under conditions effective for said antibody to specifically bind to said bacteria, and detecting binding between said antibody and said bacteria.

Polypeptides can be detected, visualized, determined, quantitated, etc. according to any effective method. Useful methods include, e.g., but are not limited to, immunoassays, RIA (radioimmunassay), ELISA, (enzyme-linked-immunosorbent assay), immunoflourescence, flow cytometry, histology, electron microscopy, light microscopy, in situ assays, immunoprecipitation, Western blot, etc.

Immunoassays may be carried in liquid or on biological support. For instance, a sample (e.g., blood, lumen, urine, cells, tissue, cerebral spinal fluid, body fluids, etc.) can be brought in contact with and immobilized onto a solid phase support or carrier such as nitrocellulose, or other solid support that is capable of immobilizing cells, cell particles or soluble proteins. The support may then be washed with suitable buffers followed by treatment with the detectably labeled bacteria specific antibody. The solid phase support can then be washed with a buffer a second time to remove unbound antibody. The amount of bound label on solid support may then be detected by conventional means.

A "solid phase support or carrier" includes any support capable of binding an antigen, antibody, or other specific binding partner. Supports or carriers include glass, polystyrene, polypropylene, polyethylene, dextran, nylon, amylases, natural and modified celluloses, polyacrylamides, and magnetite. A support material can have any structural or physical configuration. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads

One of the many ways in which a bacteria specific antibody can be detectably labeled is by linking it to an enzyme and using it in an enzyme immunoassay (EIA). See, e.g., Voller, A., "The Enzyme Linked Immunosorbent Assay (ELISA)," 1978, Diagnostic Horizons 2, 1-7, Microbiological Associates Quarterly Publication, Walkersville, Md.); Voller, A. et al., 1978, J. Clin. Pathol. 31, 507-520; Butler, J. E., 1981, Meth. Enzymol. 73, 482-523; Maggio, E. (ed.), 1980, Enzyme Immunoassay, CRC Press, Boca Raton, Fla.. The enzyme which is bound to the antibody will react with an appropriate substrate, preferably a chromogenic substrate, in such a manner as to produce a chemical moiety that can be detected, for example, by spectrophotometric, fluorimetric or by visual means. Enzymes that can be used to detectably label the antibody include, but are not limited to, malate dehydrogenase, staphylococcal nuclease, delta-5-steroid isomerase, yeast alcohol dehydrogenase, alpha.glycerophosphate, dehydrogenase, triose phosphate isomerase, horseradish peroxidase, alkaline phosphatase, asparaginase, glucose oxidase, .beta.-galactosidase, ribonuclease, urease, catalase, glucose-6-phosphate dehydrogenase, glucoamylase and acetylcholinesterase. The detection can be accomplished by colorimetric methods that employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible peptides through the use of a radioimmunoassay (RIA). See, e.g., Weintraub, B., Principles of Radioimmunoassays, Seventh Training Course on Radioligand Assay Techniques, The Endocrine Society, March, 1986. The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine. The antibody can also be detectably labeled using fluorescence emitting metals such as those in the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

The present invention also relates to preventing and/or treating inflammatory bowel conditions in a subject in need of, comprising administering lantibiotics, as well as other antibacterial compounds, which are produced by bacteria in the digestive tract of normal individuals. A probiotic approach can be used, where bacteria that produce these compounds are administered, instead of providing the compounds in purified forms.

As described in detail above, the microbial community of subjects with inflammatory bowel conditions is perturbed. These perturbations can have profound consequences on the health of the subject. Certain bacteria, such as *Ruminococcus sp.* produce lantibiotics that have protective and antibacterial effects on pathogenic bacteria. For example, it is shown above in Table 5 above that *R. gnavus* is reduced in subjects having Crohn's disease and ulcerative colitis. *R. gnavus* produces a lantibiotic (RumA) that is active against pathogenic bacteria. The reduction in the *R. gnavus* community in these subjects can result in the growth of deleterious bacteria (such as pathogenic bacteria) that in turn is associated with an inflammatory response. Conversely, certain bacteria associated with these inflammatory bowel conditions can produce lantibiotics that inhibit beneficial bacteria such as Lactobacillus species. By providing the lantibiotic (either in purified or as a probiotic), subjects with these conditions can be treated. Any lantibiotic produced by a bacteria described herein can be utilized to prevent and/or treat inflammatory bowel conditions. The RDP group, the representative genus, or the species of the bacteria listed in Tables 3 and 5 can be utilized for diagnostic, prognostic, and disease monitoring purposes in accordance with the present invention. For instance, an increase in a Moraxella osloensis was observed in Crohns mucosa in comparison to control mucosa. This information was obtained from a sequenced clone originating in the mucosa of a Crohns patient. Sequence searching of the RDP database Version 8.1 (Cole JR, Chai B, Marsh TL, Farris RJ, Wang Q, Kulam SA, Chandra S, McGarrell DM, Schmidt TM, Garrity GM, Tiedje JM. The Ribosomal Database Project (RDP-II): previewing a new autoaligner that allows regular updates and the new prokaryotic taxonomy. Nucleic Acids Res 2003 Jan 1;31(1):442-3) (see, e.g., world wide web at rdp8.cme.msu.edu/html) indicated that it was a member of the Pseudomonas_and_relatives RDP group, and more precise sequence analysis assigned it to the Moraxella genus. For the purposes of the present invention, the RDP group alone can be used as the indicator of disease status. Thus, in the above-mentioned example, classifying a bacteria as a member of the Pseudomonas_and_relatives RDP group (irrespective of its genus or species classification) is sufficient to indicate that the patient harboring the bacteria in their intestinal mucosa is more likely to be afflicted with Crohns disease, or to be regressing from a temporary remission. One or more groups can be used diagnostically. Therefore, with respect to the example above, determining that a patient's microbial community comprises both Pseudomonas_and_relatives and Acidovorax_Group bacteria indicates the existence of Crohns disease. Similar analysis can be made for all the RDP groups disclosed in Tables 3 and 5. Although not all permutations may be disclosed in the application, they can be routinely chosen from Tables 3, 5, and the appended claims.

For any given clone isolated from a subject suspected of having Crohns, Ulcerative colitis, or Pouchitis, a SSU rRNA sequence 97% identity to a known species is generally sufficient for it to be classified as that species. Similarly, about 95% identity is generally sufficient for genus and RDP group classification. Identity was determined using the BLAST algorithm (Tatusova, T. A., & Madden, T. L. (1999). BLAST 2 Sequences, a New Tool for Comparing Protein and Nucleotide Sequences. FEMS Microbiology Letters, 174, 247-250; Altschul, S. F., Madden, T. L., Schaffer, A. A., Zhang, J., Zhang, Z., Miller, W., et aI. (1997). Gapped BLAST and PSI-BLAST: a new generation of protein database search programs. Nucleic acids research, 1997 Sep 1, 25(17):3389-402; Wheeler, D. L., Chappey, C., Lash, A. E., Leipe, D. D., Madden, T. L., Schuler, G. D., et al. (2000). Database resources of the National Center for Biotechnology Information. Nucleic Acids Res, 28(1), 10-14.).

The present invention provides methods diagnosing, prognosticating, and/or monitoring disease progression of Crohn's disease ulcerative colitis, or pouchitis, or in a subject, comprising: contacting a colonic mucosal tissue sample comprising nucleic acid with a polynucleotide probe which is specific for at least one bacteria under conditions effective for said probe to hybridize specifically with said nucleic acid, and detecting hybridization between said probe and said nucleic acid, wherein an increase, as compared to a normal mucosa sample, of one or more bacteria species or RDP group selected from the following indicates the disease presence or the disease status: a) Crohn's disease: *Morexella sp.* of Pseudomonas group; *Comamonas sp.* of the Acidovorax_Group; or *Cryseobacterium sp.* of the Cytophaga_Group_I (where the RDP group and/or genus and/or genus_species can be used); b) ulcerative colitis: *Morexella sp.* of Pseudomonas_and_Relatives; *Comamonas sp.* of the Acidovorax_Group; *Clostridium sp.* of the Clostridium botulinum_Group; or *Enterococus sp.* of the Enterococcus_Group (where the RDP group and/or genus and/or genus_species can be used); or c) pouchitis (compared to normal pouch): *Ruminococus sp.* of Clostridium_Coccoides_Group; *Escherichia coli* and *Shigella sp.* of the Enterics and Relatives group; or *Fusobacterium sp.* of the Fusobacteria_Group (where the RDP group and/or genus and/or genus_species can be used).

The present invention also provides methods for diagnosing, prognosticating, and/or monitoring disease progression of Crohn's disease, ulcerative colitis, or pouchitis, comprising: contacting a colonic inucosal tissue sample comprising nucleic acid with a polynucleotide probe which is specific for at least one bacteria under conditions effective for said probe to hybridize specifically with said nucleic acid, and detecting hybridization between said probe and said nucleic acid, wherein a decrease, as compared to a normal mucosa sample, of one or more bacteria selected from the following group said bacteria indicates the disease presence or the disease status: a) Crohn's disease: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); b) ulcerative colitis: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); c) pouchitis: *Bacteroides sp.* of the Bacteroides Group; *Propionibacterium sp.* of the Propionibacterium Group (where the RDP group and/or genus and/or genus_species can be used).

The present invention also provides methods for diagnosing, prognosticating, and/or monitoring disease progression of Crohn's disease ulcerative colitis, or pouchitis, or in a subject, comprising: determining the presence of one or more of the following bacteria in a colonic mucosal tissue from a subject having Crohn's disease, ulcerative colitis, or pouchitis: a) Crohn's disease: *Morexella sp.* of Pseudomonas group; *Comamonas sp.* of the Acidovorax_Group; or *Ctyseobacterium sp.* of the Cytophaga_Group_I (where the RDP group and/or genus and/or genus_species can be used); b) ulcerative colitis: *Morexella sp.* of Pseudomonas_and_Relatives; *Comamonas sp.* of the Acidovorax_Group; *Clostridium sp.* of the Clostridium botulinum_Group; or *Enterococus sp.* of the Enterococcus_Group (where the RDP group and/or genus and/or genus_species can be used); c) pouchitis (compared to normal pouch): Ruminococus sp. of Clostridium_Coccoides_Group; *Eschericlzia coli and Shigella sp.* of the Enterics and Relatives group; or *Fusobacterium sp.* of the Fusobacteria_Group (where the RDP group and/or genus and/or genus_species can be used).

The present invention also provides methods for diagnosing, prognosticating, and/or monitoring disease progression of Crohn's disease, ulcerative colitis, or pouchitis, comprising: determining the absence of one or more of the following bacteria in a colonic mucosal tissue from a subject having Crohn's disease, ulcerative colitis, or pouchitis: a) Crohn's disease: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); b) ulcerative colitis: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); c) pouchitis: *Bacteroides sp.* of the Bacteroides Group; or *Propionibacterium sp.* of the Propionibacterium Group (where the RDP group and/or genus and/or genus_species can be used).

The present invention also provides method for diagnosing, prognosticating, and/or monitoring disease progression of Crohn's disease or ulcerative colitis, in a subject, comprising: contacting a lumen sample comprising nucleic acid with a polynucleotide probe which is specific for at least one bacteria under conditions effective for said probe to hybridize specifically with said nucleic acid, and detecting hybridization between said probe and said nucleic acid, wherein an increase, as compared to a normal lumen sample, of one or more bacteria selected from the following indicates the disease presence or the disease status: a) Crohn's disease: *Bacteriodes sp.* of the Bacteriodes_Group; or *Chryseobacterium sp.* of the Cytophaga_Group_I (where the RDP group and/or genus and/or genus_species can be used); or b) ulcerative colitis: *Bacteriodes sp.* of the Bacteriodes_Group; or *Cluyseobacterium sp.* of the cytophaga_Group_I (where the RDP group and/or genus and/or genus_species can be used).

The present invention also provides methods for diagnosing, prognosticating, and/or monitoring disease progression of Crohn's disease or ulcerative colitis in a subject, comprising: contacting a lumen sample comprising nucleic acid with a polynucleotide probe which is specific for at least one bacteria under conditions effective for said probe to hybridize specifically with said nucleic acid, and detecting hybridization between said probe and said nucleic acid, wherein a decrease, as compared to a normal lumen sample, of *Acinetobacter sp.* or *Moraxella sp.* of the Pseudomonas and relatives group indicates that said subject has Crohn's disease or ulcerative colitis (where the RDP group and/or genus and/or genus_species can be used).

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

### EXAMPLES

Sample Collection and DNA extraction: Endoscopic mucosal tissue samples were collected from the terminal ileum, cecum+ascending colon, transverse colon, sigmoid colon and the rectum of patients with IBD and Pouchitis as well as healthy controls undergoing the colonoscopy. Some of the tissue samples were washed in saline prior to analysis to remove non-adherent bacteria (washed vs. unwashed samples). Retained lumen samples were also collected via the endoscope at the time of procedure. The samples were fingerprinted for bacterial patterns in 4 control, 2 UC, 4 CD and 3 patients with pouchitis, and 5 patients with pouch without pouchitis using the ALH methodology. The DNA extractions were performed using the Bio101 soil kit from Qbiogene, Inc, Montreal, Quebec according to the manufacturers instructions. These ALH amplicons were pooled, then cloned and sequenced to identify the bacterial components that were indicative of the disease state.

Amplicon Length Heterogeneity ALH Fingerprinting: ALH is a technique of bacterial fingerprinting see Ritchie, N.J., et al., Use of Length Heterogeneity PCR and Fatty Acid Methyl Ester Profiles to Characterize Microbial Communities in Soil. Applied and Environmental Microbiology, 2000. 66(4): p. 1668-1675; Suzuki, M., M.S. Rappe, and S.J. Giovannoni, Kinetic bias in estimates of coastal picoplankton community structure obtained by measurements of small-subunit rRNA gene PCR amplicon length heterogeneity. Applied and Environmental Microbiology [Appl. Environ. Microbiol.]. ALH is a PCR-based analysis which can distinguish different organisms based on natural variations in the length of 16S ribosomal DNA sequences. Purified DNA (10ng) was amplified with PCR by using a fluorescently- labeled forward primer 27F (5'-[6FAM] AGAGTTTGATCCTGGCTCA G-3') and unlabeled reverse primer 338R' (5'-GCTGCCTCCCGTAGGAGT-3'). Both primers are highly specific for eubacteria. Alternatively, we have primers that amplify the corresponding region in Archae see Burggraf, S., T. Mayer, R. Amann, S. Schadhauser, C.R. Woese and K.O. Stetter, Identifying Members of the Domain Archaea with rRNA-Targeted Oligonicleotide Probes. App. Environ. Microbiol., 1994. 60: p. 3112-3119.. We have recently optimized primers specific to the ITS of fungi and demonstrated that these region of the rRNA operon is more informative that the SSU rRNA see Borneman, J. and J. Hartin, PCR primers that amplify fungal rRNA genes from Environmental Samples. App. Environ. Microbiol., 2000. 66(10): p. 4356-4360.. The reactions were performed using 50-ul (final volume) mixtures containing 1 X PCR buffer, 0.6% bovine serum albumin, 1.5 mM MgCl2, each deoxynucleoside triphosphate at a concentration of 0.2mM, each primer at a concentration of 0.2 uM, and 2 U of *Taq* DNA polymerase. Initial denaturation at 94 C for 3 min was followed by 25 cycles consisting of denaturation at 94 C for 45 sec, annealing at 55 C for 45 s, and extension at 72 C for 2 min. There was a final extension step that consists of 72 C for 7 min. ALH samples are were stored at -20 C in the dark until used (usually less than 1 week).

The ALH PCR products were separated on the SCE9610 capillary fluorescent sequencer (Spectrumedix LLC, State College, PA) and analyzed with their GenoSpectrum software package. The software converts fluorescence data into electropherograms. The peaks of the electropherograms represent different populations of microflora of different sizes. All fingerprinting data was analyzed using software (Interleave 1.0, BioSpherex LLC) that combines data from several runs, interleaves the various profiles, normalizes the data, and calculates diversity indices. The normalized peak areas were calculated by dividing an individual peak area by the total peak area in that profile.

Analysis of ALH Fingerprints: The diversity of ALH fingerprints were analyzed using indices of Richness (R), Evenness (E) and the Shannon-Weaver diversity index (SW) in groups comparing IBD to controls, see Hughes, j.B., et al., Counting the Uncountable: Statistical Approaches to Estimating Microbial Diversity. App. Environ. Microbiol., 2001. 67(10): p. 4399-4406.. IBD related parameters such as disease activity, and histologically involved and uninvolved parts of the ileum & colon were compared using the diversity indices.

These fingerprints were analyzed to determine global clustering of ALH fingerprints into presence or absence of IBD, IBD types (CD, UC and pouchitis), disease activity, and involved and uninvolved parts of the ileum & colon (tissue state). Multidimensional Reductions Analysis [Principal Component Analysis (PCA), Principal Coordinate Analysis (PCO), Canonical Correspondence Analysis (CCA)] and Clustering Analysis was done using the Multi Variate Statistical Package (MVSP), Kovach Computing Services, Wales, UK. The following analyses will be done. Generation of dendograms by Unweighted Pair Group Method using Arithmetic Averages (UPGMA).

*Principal Component Analysis (PCA):* PCA is one of the best known and earliest ordination methods, first described by Karl Pearson (1901). Graphically, it is a rotation of a swarm of data points in multidimensional space so that the longest axis (the axis with the greatest variance) is the first PCA axis, the second longest axis perpendicular to the first is the second PCA axis, and so forth. The first few PCA axes represent the greatest amount of variation in the data set. The first two or three axes are generally expected to account for a large proportion of the variance, e.g. 50-60% or more.

*PrincipalCoordinates Analysis (PCG):* PCO can be viewed as a more general form of PCA. PCO can use a variety of different measures of distance or similarity. In general, the distances or similarities are measured between the cases directly, rather than the variables as in PCA. The main advantage of PCO is that many different kinds of similarity or distance measures can be used. PCO is restricted to analyzing distances or similarities that are metric and the distances used must be able to be viewed in some sensible geometrical manner e.g. a triangle.

*Canonical Corespondance Analysis (CCA):* In PCA & PCO, the data are subjected to some type of mathematical manipulation in order to reveal the most important trends. These trends are then often compared to other data relating to the same samples to determined the relationship between the two. However, in CCA, the data are directly related. CCA is a multivariate direct gradient analysis method that has become very widely used in ecology.

Cluster Analysis: Cluster analysis is a term used to describe a set of numerical techniques in which the main purpose is to divide the objects of study into discrete groups. These groups are based on the characteristics of the objects and it is hoped the clusters will have some sort of significance related to the research questions being asked. Cluster analysis is used in many scientific disciplines and a wide variety of techniques have been developed to suit different types of approaches. The most commonly used ones are the agglomerative hierarchical methods. Hierarchical methods arrange the clusters into a hierarchy so that the relationships between the different groups are apparent and the results are presented in a tree-like diagram called a dendrogram. The agglomerative methods used to create a dendrogram start by successively combining the most similar objects until all are in a single, hierarchical group. Similarly dendograms can be created using the well established Unweighted Pair Group Method using Arithmetic Averages (UPGMA) and K-means.

Putative ALH fingerprint patterns (i.e. presence or absence of certain amplicon peaks) associated with IBD presence, disease types, disease activity, and tissue state were identified. For this purpose, we will visually inspect histograms of ALH fingerprints. To determined statistical correlations of peaks to IBD related variables, we also used multivariate analysis for large variable sets i.e. discriminate analysis and Canonical correspondence analysis. We also employed computerized data mining tools with supervised and unsupervised pattern recognition algorithms. These include C4.5, support vector machines, and self organizing maps. Hence, these analyses will be used to determine if ALH fingerprinting can distinguish between IBD related parameters (disease presence, type, activity, tissue state) and determine particular ALH patterns (presence or absence of a peak or sets of peaks) associated with IBD.

Sequencing of ALH Clones: The PCR product generated with primers used for ALH fingerprinting were cloned by using pGEM-T Easy Vector System II (Promega Corp., Madison, WI). Clones were screened assessing for inserts using alpha-complementation with X-Gal (5 bromo-4chloro-3indoyl-B-D-galactopyranoside) and IPTG (isopropyl-B-D-thiogalactopyranoside). Inserts were sequenced by using *Taq* dye terminator chemistry and the sequencing products were separated on a SCE9610 fluorescent capillary sequencer.

Analysis of ALH clone data: The above ALH clone sequences were compared to sequences in the RDP database to assess for patterns of microflora using a novel program (CloneID 1.0, BioSpherex LLC). The algorithm basically uses Megablast to compare the clone sequence data to the RDP database and compiles a table using the RDP numbers to correlate the identification with a hierarchical classification scheme. These same ALH clones were fingerprinted to determine the empirical ALH size and correlated with the original ALH fingerprint of sample using a second program (CloneMatch 1.0, BioSpherex, LLC).

### Results of Crohns (CD) and Ulcerative Colitis (UC) Analysis

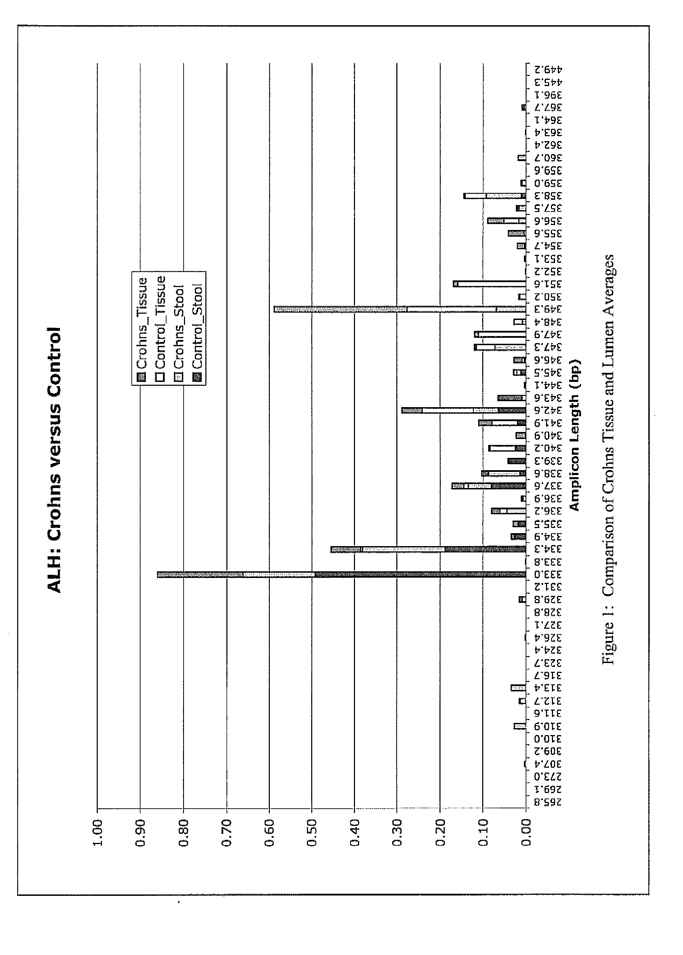

Although ALH fingerprints vary qualitatively and quantitatively between individuals, there are very distinct diagnostic patterns that can be seen from the analysis of pooled tissue (mucosa) and lumen samples. Figure 1 is a histogram compiled from the average of the ALH fingerprint from all the Crohns samples and Control samples (i.e. all individuals and all locations) showing amplicon lengths in base pairs (bp) on the x-axis and relative abundances on the y-axis. The pooled Controls Tissue samples (white bars) had very distinct ALH profile that differed dramatically from the Controls Lumen samples (black bars) indicating that there is a distinct microflora community adhering to the mucosa as a biofilm. In contrast there was not a clear differentiation between the lumen and tissue microflora in Crohns disease indicating a dramatic dysbiosis in which many of the bacterial species normally in lumen are found in the biofilm. Thus, there was much more overlap between the ALH amplicons of Crohns Tissue (light grey bars) and Crohns Lumen (dark grey bars) with Control Lumen (black bars). There are diagnostic ALH amplicons that occur predominantly in the Control Lumen samples and Crohns tissue (i.e. at 333.0 bp, 334.3 bp, and 338.6). Furthermore, there are some ALH amplicons that are unique for Crohns tissue (i.e. 310.9 bp and 313.4 bp) but on average they make up a small proportion of the microflora community.

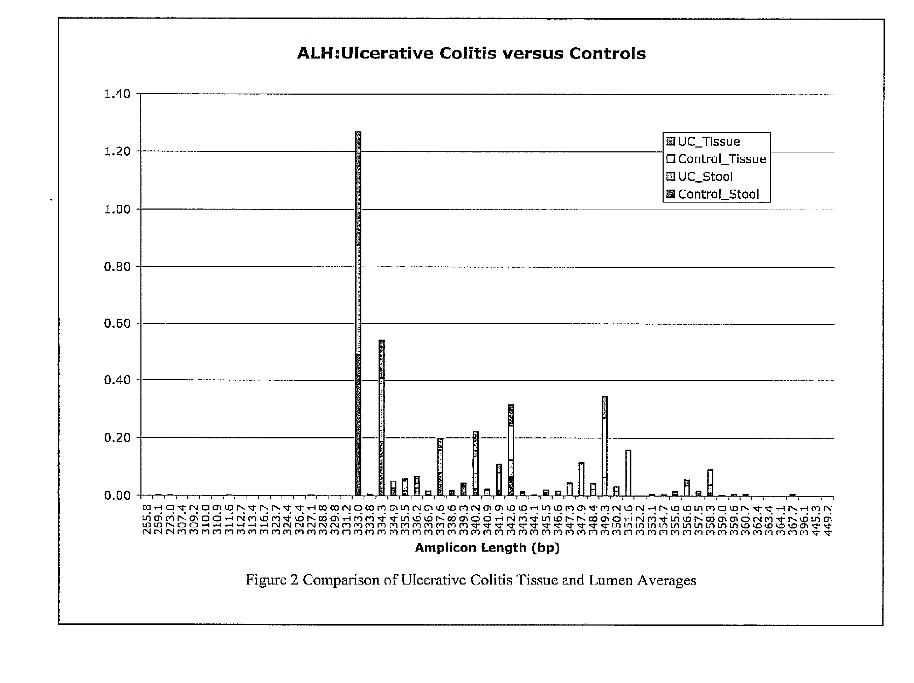

Similarly, there seems to be dysbiosis in Ulcerative colitis (UC) as the ALH profiles of UC Tissue and UC Lumen are similar to Control Lumen with the ALH profile of the Controls being very distinct (Figure 2). Thus, there was much more overlap between the ALH amplicons of UC Tissue (light grey bars) and UC Lumen (dark grey bars) with Control Lumen (black bars). Some of the diagnostic ALH amplicons that were observed in CD (see above) are the same amplicons that are diagnostic in UC (i.e. at 333.0 bp, 334.3 bp, and 338.6 bp). Furthermore, there are distinct ALH amplicons that occur only in the UC tissue (i.e. 334.6 bp).

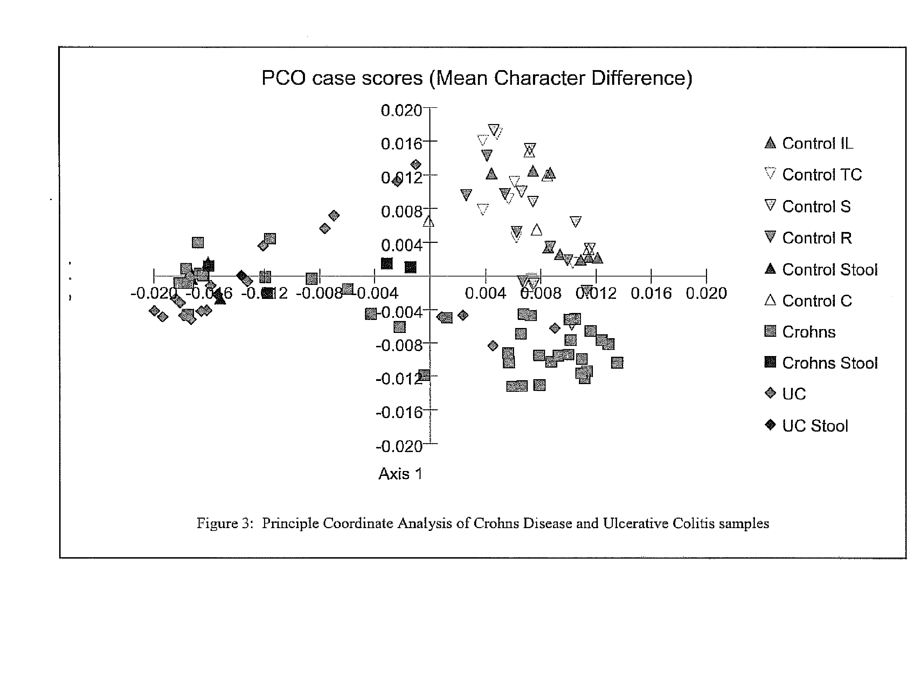

When the mean character differences for ALH profiles from Controls, CD, and UC were examined using Principle Coordinate Analysis (PCO), dramatic clustering patterns can be seen for UC and CD that is distinct from the Control samples (Figure 3). We clearly see distinct clustering of Control ALH profiles in the 1^{st} quadrant, UC clusters in the 3^{rd} & 4^{th} quadrants boundary, and CD is mainly clustered in the 2^{nd} quadrant. It is also important to note that the lumen samples cluster in the 3^{rd} & 4^{th} quadrant associated with UC. There are also several Crohns ALH profiles that cluster in this 3^{rd} & 4^{th} quadrants suggesting that there is variation in the tissue microflora of Crohns and that, in specific samples, these ALH profiles are similar to those of UC.

PCA and Canonical Correspondence Analysis demonstrates a similar clustering of healthy controls separate from CD and UC patients. The dendograms produced with UPGMA clustering using a Jacard distance measure also show the same general patterns as the PCO analysis.

We have cloned and sequenced pooled ALH amplicons from the UC, CD and healthy controls samples and these sequence data were used to identify the bacterial species associated with each disease state. Table 1 summarizes the key bacterial groups based on the RDP classification scheme that occur at a frequency of greater than 5% of the microfloral community. The data supports the ALH profiles in that the microflora found on the mucosal surface of CD and UC tissue resemble the microfloral composition of lumen in healthy individuals and that this composition differs from the microfloral composition of the controls mucosa. Specifically, members of the Pseudomonads such as *Moraxella sp.* and members of the Acidovorax group such as *Comoamonas sp.* are associated with Control lumen, CD lumen, CD mucosa, UC lumen, and UC mucosa. Additionally, members of the Cyotphaga group such as Chryseobacterium balustinum are associated with CD mucosa, CD lumen, and UC lumen. Finally, members of both of the Clostridium group (*Clostridium paraputrificum*) and Enterococcus (*Enterococcus hirae*) are also associated with UC mucosa. We also note that there is a quantitative decrease in the Bacteroides group in UC mucosa and CD mucosa compared to the Control mucosa. In summary, we conclude that there are bacterial species that are associated in the CD biofilm and UC biofilm that are normally found in lumen and that this indicates severe dysbiosis.

### Results for Pouchitis Analysis

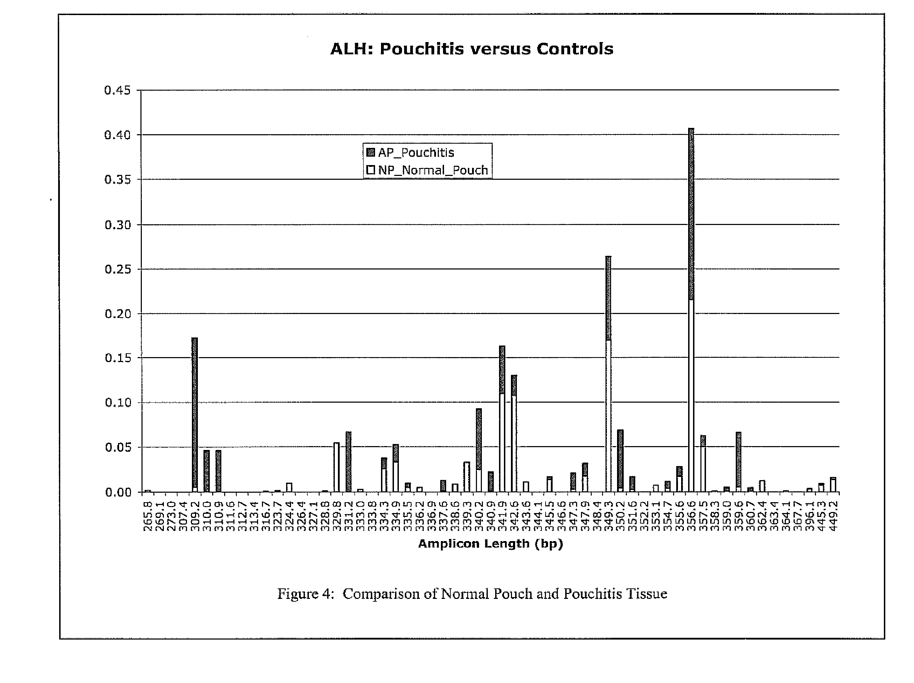

Figure 4 is a histogram compiled from the average of the ALH fingerprint from all the Pouchitis samples (AP) and Normal pouch samples (NP), that is samples from patients with active Pouchitis (AP) and patients with a Pouch but are normal upon examination (NP). As seen in CD and UC, the pooled NP mucosa samples (white bars) had very distinct ALH profile that differed dramatically from the NP mucosa samples (black bars) indicating that there is a distinct microflora community adhering to the mucosa as a biofilm. Furthermore, the ALH amplicon profiles from the NP samples were different that healthy control patients that did not have a Pouch. There are diagnostic ALH amplicons that occur predominantly in the AP mucosa samples (i.e. at 309.2 bp, 310.0 bp, 310.9 bp, 331.2 bp, 350.2 bp, and 359.6 bp) that are not the predominant diagnostic ALH amplicons in CD and UC. Thus, the dysbiosis in Pouchitis seems to be very different from CD and UC and may involve different pathology. Furthermore, the actual components of the community in the disease state vary from individual to individual. For example, the ALH amplicons at 309.2 bp, 310.0 bp, 310.9 bp are major components in one patient but are only minor components of others. Importantly, the Normal Pouch patients have abnormal microflora content in the mucosal biofilm and these patients may be continuously in a semi-disease state.

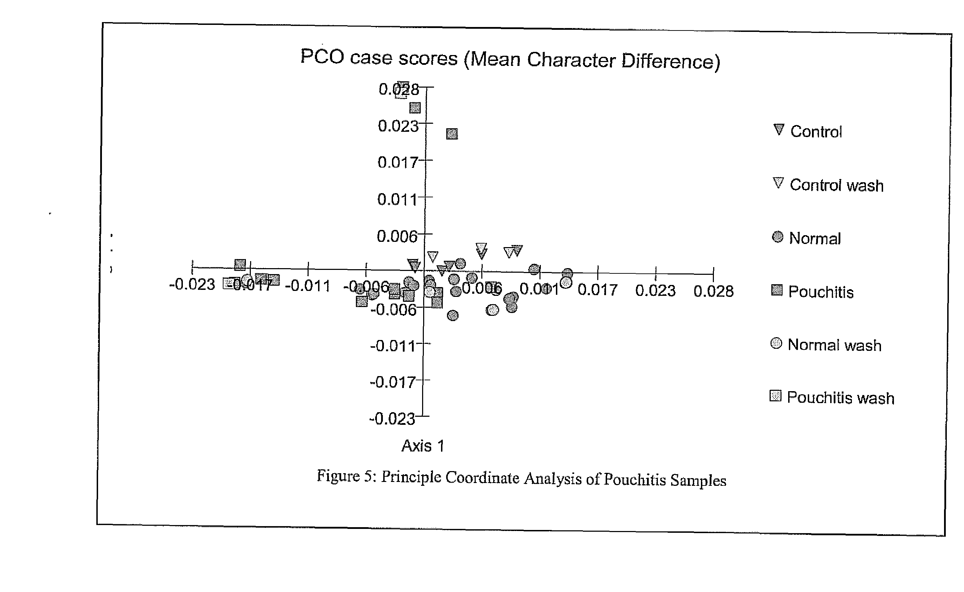

When the mean character differences for ALH profiles from NP and AP samples were examined using Principle Coordinate Analysis (PCO), a general clustering pattern can be seen for NP in the center of the graph that is distinct from three clusters of AP samples (Figure 5). Interestingly, each of these AP clusters are from separate patient confirming that patients with Pouchitis exhibit much more variation in the microflora in the mucosal biofilm. It should be noted that the separate cluster found on the Y axis above the cluster of Normal Pouch is the patient that displayed the distinct ALH amplicons at 309.2 bp, 310.0 bp, 310.9 bp. The extent of activity of the disease may be reflected in the extent of dysbiosis depicted in the PCO plot. Furthermore, the pattern is consistent whether the samples have been washed or not washed in saline as is the case in the CD and UC samples.

We have cloned and sequenced pooled ALH amplicons from the NP and AP mucosal samples and these sequence data were used to identify the bacterial species associated with each disease state. Table 1 summarizes the key bacterial groups based on the RDP classification scheme that occur at a frequency of greater than 5% of the microfloral community. The data supports the ALH profiles in that the microflora found on the mucosal surface of both AP and NP tissue are different from that found in healthy individuals and these do not reflect the microflora found in Normal lumen as found in CD and UC. Specifically, members of the Clotridium group (i.e. *Clostridium paraputrificum*), members of Enterics (i.e. *E.coli and Shigella sp*.), and members of the Streptococcus group (i.e. *Streptococcus brevis*) are found associated with the mucosa of NP patients. On the other hand, the microflora associated with the mucosa in AP patients was very diverse and differed from the NP patients. Specifically, we observed that members of the Enterics (i.e. *E.coli and Shigella sp*.) and Fusobacterium group (i.e. *Fusobacterium varium*) was associated with the mucosa in the AP patients and that there was a dramatic loss of members of the Streptococci group (i.e. *Streptococcus brevis*). Furthermore, a different *Ruminococcus* species (*Ruminocaccus obeum*) was associated with AP patients but it is not clear that this strain difference would contribute to the pathology. In summary, it looks like both NP and AP patients have dysbiosis compared to the normal controls and that there is a dramatic loss of Streptococci in AP patients. Furthermore, there seems to be patient specific (see Figure 5) ALH fingerprints suggesting significant variation in the microflora between patients.

### Correlation of ALH Amplicons and Microflora

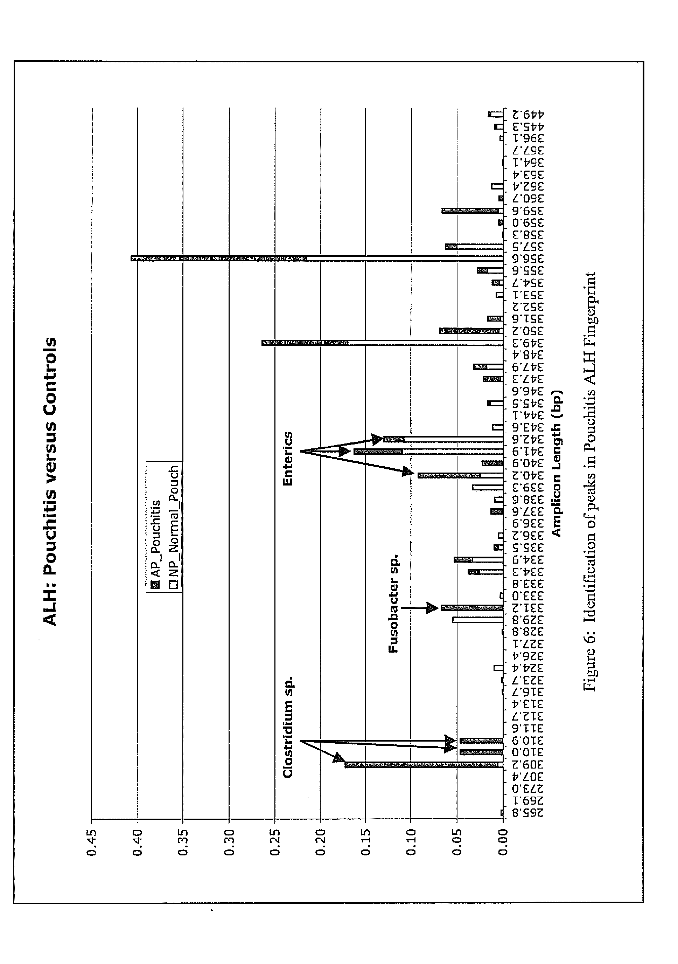

We have correlated the experimentally determinedALH amplicon size of clones with the identifications obtained from sequencing these. For example, we have labeled the main amplicons in the histogram Pouchitis and Normal Pouch ALH fingerprints in Figure 6. We then use this information to correlate what bacterial species are in the diagnostic peaks of the ALH profiles.

Hence, the invention particularly relates to the following aspects:
1. A method for diagnosing, prognosticating, and/or monitoring disease progression of Crohn's disease ulcerative colitis, or pouchitis, or in a subject, comprising:
   contacting a colonic mucosal tissue sample comprising nucleic acid with a polynucleotide probe which is specific for at least one bacteria under conditions effective for said probe to hybridize specifically with said nucleic acid, and
   detecting hybridization between said probe and said nucleic acid,
   wherein an increase, as compared to a nonnal mucosa sample, of one or more bacteria selected from the following indicates the disease presence or the disease status:
      a) Crolm's disease: *Morexella sp.* of Pseudomonas group; *Comamonas sp.* of the Acidovorax_Group; or *Cryseobactervum sp.* of the Cytophaga_Group_I;
      b) ulcerative colitis: *Morexella sp.* of Pseudomonas_and_Relatives; *Comamonas sp.* of the Acidovorax_Group; *Clostridium sp.* of the Clostridium botulinum_Group; or *Enterococus sp.* of the Enterococcus_Group;
      c) pouchitis (compared to normal pouch): *Ruminococus sp.* of Clostridium_Coccoides_Group; *Escherichia coli* and *Shigella sp.* of the Enterics and Relatives group; or *Fusobacterium sp.* of the Fusobacteria_Group.
2. A method for diagnosing, prognosticating, and/or monitoring disease progression of Crohn's disease, ulcerative colitis, or pouchitis, comprising:
   contacting a colonic mucosal tissue sample comprising nucleic acid with a polynucleotide probe which is specific for at least one bacteria under conditions effective for said probe to hybridize specifically with said nucleic acid, and
   detecting hybridization between said probe and said nucleic acid,
   wherein a decrease, as compared to a normal mucosa sample, of one or more bacteria selected from the following group said bacteria indicates the disease presence or the disease status:
      a) Crohn's disease: *Bacteroides sp.* of the Bacteroides Group; *Propionibacterium sp.* of the Propionibacterium Group; or *Ruminoccocus sp.* of the Clostridium_Coccoides Group;
      b) ulcerative colitis: *Bacteroides sp.* of the Bacteroides Group; *Propionibacterium sp.* of the Propionibacterium Group; or *Ruminoccocus sp.* of the Clostridiuim_Coccoides Group;
      c) pouclxitis: *Bacteroides sp.* of the Bacteroides Group; *Propionibacterium sp.* of the Propionibacterium Group.
3. A method for diagnosing, prognosticating, and/or monitoring disease progression of Crohn's disease ulcerative colitis, or pouchitis, or in a subject, comprising:
   determining the presence of one or more of the following bacteria in a colonic mucosal tissue from a subject having Crohn's disease, ulcerative colitis, or pouchitis:
      a) Crohn's disease: *Moraxella sp.* of Pseudomonas group; *Conianionas sp.* of the Acidovorax_Group; or *Cryseobacterium sp.* of the Cytophaga_Group_I;
      b) ulcerative colitis: *Morexella sp.* of Pseudomonas_and_Relatives; *Comamonas sp.* of the Acidovorax_Group; *Clostridium sp.* of the Clostridium botulinum_Group; or *Enterococus sp.* of the Enterococcus_Group;
      c) pouchitis (compared to normal pouch): Ruminococus sp. of Clostridium_Coccoides_Group; *Escherichia coli and Shigella sp.* of the Enterics and Relatives group; or *Fusobacterium sp.* of the Fusobacteria_Group.
4. A method of any of aspects 1-3, wherein the presence of said bacteria is determined using an antibody specific for said bacteria.
5. A method of any of aspects 1-3, wherein the presence of said bacteria is determined using an oligonucleotide probe specific for said bacteria.
6. A method of any of aspects 1-3, wherein the presence of said bacteria is determined using nucleic acid fingerprinting, PCR, nucleotide sequencing, Southern blot, and/or DNA microarrays comprising a plurality of sequences specific for one or more of said bacteria.
7. A method of any of aspects 1-3, wherein said bacteria is increased in comparison to a control sample.
8. A method for diagnosing, prognosticating, and/or monitoring disease progression of Crohn's disease, ulcerative colitis, or pouchitis, comprising:
   determining the absence of one or more of the following bacteria in a colonic mucosal tissue from a subject having Crohn's disease, ulcerative colitis, or pouchitis:
      a) Crohn's disease: *Bacteroides sp.* of the Bacteroides Group; *Prapionibacterium sp.* of the Propionibacterium Group; or *Ruminoccocus sp.* of the Clostridium_Coccoides Group;
      b) ulcerative colitis: *Bacteroides sp.* of the Bacteroides Group; *Propionibacterium sp.* of the Propionibacterium Group; or *Runainoccocus sp.* of the Clostridium_Coccoides Group;
      c) pouchitis: *Bacteroides sp.* of the Bacteroides Group; or *Propionibacterium sp.* of the Propionibacterium Group.
9. A method of aspect 8, wherein the absence of said bacteria is determined using an antibody specific for said bacteria.
10. A method of aspect 8, wherein the absence of said bacteria is determined using an oligonucleotide probe specific for said bacteria.
11. A method of any one of aspects 8-10, wherein the absence of said bacteria is determined in comparison to a control sample.
12. A method for diagnosing, prognosticating, and/or monitoring disease progression of Crohn's disease or ulcerative colitis, in a subject, comprising:
   contacting a lumen sample comprising nucleic acid with a polynucleotide probe which is specific for at least one bacteria under conditions effective for said probe to hybridize specifically with said nucleic acid, and
   detecting hybridization between said probe and said nucleic acid,
   wherein an increase, as compared to a normal lumen sample, of one or more bacteria selected from the following indicates the disease presence or the disease status:
      a) Crohn's disease: *Bacteriodes sp.* of the Bacteriodes_Group; or *Chryseabacterium sp.* of the Cytophaga_Group_I; or
      b) ulcerative colitis: *Bacteriodes sp.* of the Bacteriodes_Group; or *Chryseobacterium sp.* of the Cytophaga_Group_I.
13. A method for diagnosing, prognosticating, and/or monitoring disease progression of Crohn's disease or ulcerative colitis in a subject, comprising:
   contacting a lumen sample comprising nucleic acid with a polynucleotide probe which is specific for at least one bacteria under conditions effective for said probe to hybridize specifically with said nucleic acid, and
   detecting hybridization between said probe and said nucleic acid,
   wherein a decrease, as compared to a normal lumen sample, of *Acinetobacter sp.* or *Moraxella sp.* of the Pseudomonas and relatives group indicates that said subject has Crohn's disease or ulcerative colitis.
14. A method for diagnosing, prognosticating, and/or monitoring disease progression of a polymicrobial disease, comprising:
   performing an amplicons length heterogeneity reaction on a sample comprising nucleic acid with at least two polynucleotide probe primers which are effective for amplifying the microbial community present in said sample, and
   detecting the reaction products of said amplification reaction, whereby said reaction products comprise a pattern that indicate the presence of a polymicrobial disease or its disease status.
15. A method of aspect 14, wherein said disease is an inflammatory bowel disease.
16. A method of aspect 14, wherein said primers amplify regions of the SSU rRNA.
17. A method of aspect 14, wherein said primers amplify regions of the LSU rRNA.
18. A method of aspect 14, wherein at least one primer is specific for a variable region of SSU or LSU rRNA.
19. A method of aspect 14, wherein at least one primer is specific for a helix region of SSU or LSU rRNA.
20. A method of aspect 14, wherein said primers amplify a variable region of SSU or LSU rRNA.
21. A method of aspect 20, wherein said primers are:
   27F (5'-[6FAM] AGAGTTTGATCCTGGCTCAG-3') (SEQ ID NO: 37), and
   338R' (5'-GCTGCCTCCCGTAGGAGT-3) (SEQ ID NO: 38).
22. A method of aspect 14, wherein the disease is an inflammatory bowel disease, and further comprising performing PCO analysis to determine which reaction products indicate the presence of ulcerative colitis, pouchitis, or Crohn's disease.
23. A method of aspect 14, further comprising sequencing said reaction products.
24. A method of aspect 14, further comprising sequencing said reaction products and correlating said sequence with a microbe type.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

**Table 1 Increase or Decrease of ALH Fingerprint amplicons greater than 5% in Crohns mucosa versus Control Mucosa**

| Amplicon Size (bp) | 333.0 | 334.3 | 336.2 | 337.6 | 338.6 | 340.2 | 341.9 | 342.6 | 343.6 | 347.3 | 347.9 | 349.3 | 351.6 | 358.3 |
|---|---|---|---|---|---|---|---|---|---|---|---|---|---|---|
| Control Mucosa | | | 2% | 1% | | 6% | 6% | 12% | 1% | 4% | 11% | 21% | 16% | 5% |
| Control Mucosa | 20% | 7% | 2% | 3% | 2% | | 3% | 5% | 6% | | 1% | 31% | 1% | |
| | | | | | | | | | | | | | | |
| Increased in Crohns mucosa | 333.0 | 334.3 | | | 338.6 | | | | 343.6 | | | | | |
| Decreased in Crohns mucosa | | | | | | 340.2 | 341.9 | 342.6 | | 347.9 | 347.9 | 349.3 | 351.6 | 358.3 |

**Table 2 Increase or Decrease of ALH Fingeprint amplicons greater than 5% in Uncerative Colitis mucosa versus Control Mucosa**

| Amplicon Size (bp) | 333.0 | 334.3 | 340.2 | 341.9 | 342.6 | 347.9 | 349.3 | 351.6 | 358.3 |
|---|---|---|---|---|---|---|---|---|---|
| Control Mucosa | | | 6% | 6% | 12% | 11% | 21% | 16% | 5% |
| UC Mucosa | 39% | 13% | 9% | 3% | 7% | | 7% | | |
| | | | | | | | | | |
| Increased in UCmucosa | 333.0 | 334.3 | 340.2 | | | | | | |
| Decreased in UCmucosa | | | | 341.9 | 342.6 | 347.9 | 349.3 | 351.6 | 358.3 |

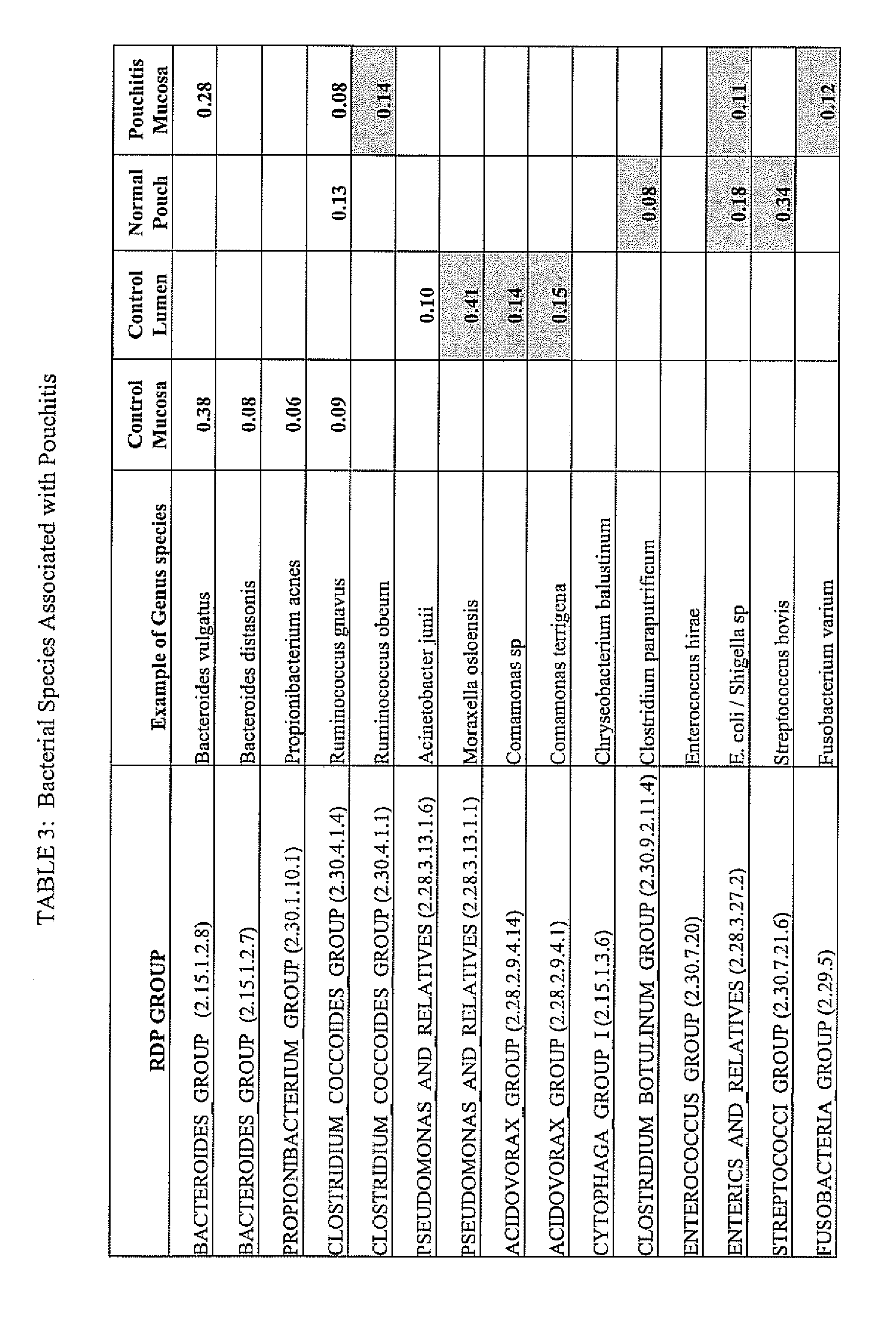

**Table 4 Increase or Decrease of ALH Fingerprint amplicons greater than 5% in Pouchitis mucosa versus Normal Pouch**

| Amplicon Size (bp) | 309.2 | 310.0 | 310.9 | 329.8 | 331.2 | 340.2 | 341.9 | 342.6 | 349.3 | 350.2 | 356.6 | 357.5 | 359.6 |
|---|---|---|---|---|---|---|---|---|---|---|---|---|---|
| Normal Pouch | 1% | | | 5% | | 2% | 11% | 11% | 17% | | 22% | 5% | 1% |
| Pouchitis | 17% | 5% | 5% | | 7% | 7% | 5% | 2% | 9% | 6% | 19% | 1% | 6% |
| | | | | | | | | | | | | | |
| Increased in Pouchitis | 309.2 | 310.0 | 310.9 | | 331.2 | 340.2 | | | | 350.2 | 356.6 | | 359.6 |
| Decreased in Pouchitis | | | | 329.8 | | | 341.9 | 342.6 | 349.3 | | | 357.5 | |

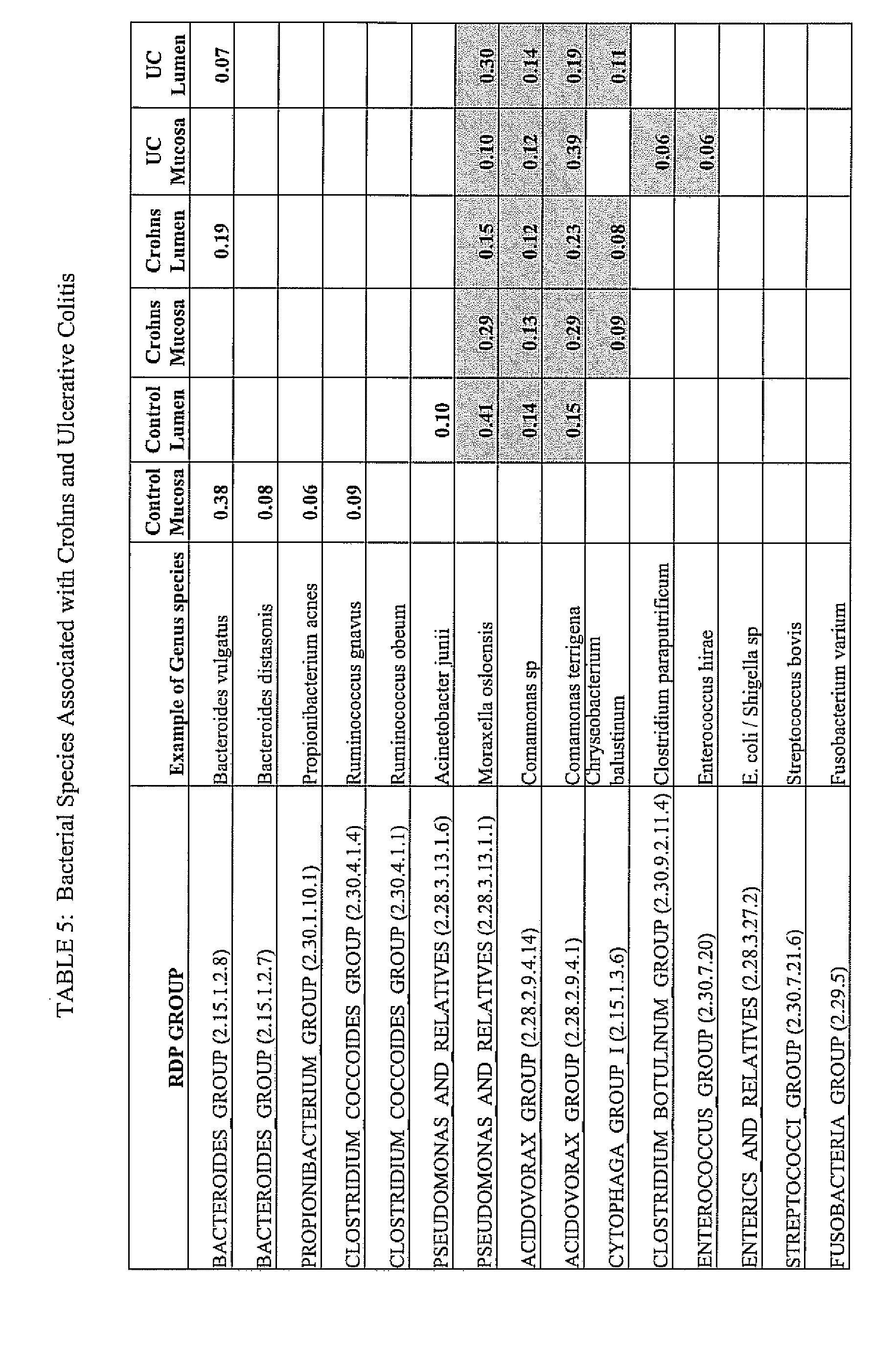

**TABLE 6**

| **RDP REFERENCE** | **SEQ ID NO** | **GENUS SPECIES** |
|---|---|---|
| 2.28.3.13.1.6 | 14 | Acinetobacter junii |
| 2.15.1.2.7 | 1 | Bacteroides distasonis |
| 2.15.1.2.8 | 2 | Bacteroides fragilis |
| 2.15.1.2.8 | 3 | Bacteroides ovatus |
| 2.15.1.2.8 | 4 | Bacteroides vulgatus |
| 2.15.1.3.6 | 17 | Bergeyella zoohelcum |
| 2.15.1.3.6 | 18 | Chryseobacterium balustinum str. SBR1044 |
| 2.15.1.3.6 | 19 | Chryseobacterium balustinum str. SBR2024 |
| 2.28.2.9.4.1 | 16 | Comamonas terrigena |
| 2.28.2.9.4.14 | 15 | Comamonas sp. |
| 2.28.3.13.1.1 | 11 | Moraxella cuniculi |
| 2.28.3.13.1.1 | 12 | Moraxella lacunata |
| 2.28.3.13.1.1 | 13 | Moraxella osloensis |
| 2.28.3.27.2 | 25 | Escherichia coli |
| 2.28.3.27.2 | 26 | Salmonella bovis |
| 2.28.3.27.2 | 27 | Shigella boydii |
| 2.28.3.27.2 | 28 | Shigella dysenteriae |
| 2.28.3.27.2 | 29 | Shigella flexneri |
| 2.29.5 | 34 | Fusobacterium alocis |
| 2.29.5 | 35 | Fusobacterium nucleatum |
| 2.29.5 | 36 | Fusobacterium valium |
| 2.30.1.10.1 | 5 | Propionibacterium acnes |
| 2.30.4.1.1 | 10 | Clostridium sp. |
| 2.30.4.1.4 | 6 | Clostridium nexile |
| 2.30.4.1.4 | 7 | Eubacterium formicigenerans |
| 2.30.4.1.4 | 8 | Ruminococcus gnavus |
| 2.30.4.1.4 | 9 | Ruminococcus torques |
| 2.30.7.20 | 21 | Enterococcus cecorum |
| 2.30.7.20 | 22 | Enterococcus columbae |
| 2.30.7.20 | 23 | Enterococcus hirae |
| 2.30.7.20 | 24 | Tetragenococcus halophilus |
| 2.30.7.21.6 | 30 | Streptococcus bovis |
| 2.30.7.21.6 | 31 | Streptococcus infantarius |
| 2.30.7.21.6 | 32 | Streptococcus salivarius |
| 2.30.7.21.6 | 33 | Streptococcus thermophilus |
| 2.30.9.2.11.4 | 20 | Clostridium paraputrificum |

## Claims

1. A method for diagnosing, prognosticating, and/or monitoring disease progression of a polymicrobial disease, comprising:
performing an amplicons length heterogeneity reaction on a sample comprising nucleic acid with at least two polynucleotide probe primers which are effective for amplifying the microbial community present in said sample, and
detecting the reaction products of said amplification reaction, whereby said reaction products comprise a pattern that indicate the presence of a polymicrobial disease or its disease status.

2. A method of claim 1, wherein said disease is an inflammatory bowel disease.

3. A method of claim 1 or, wherein said primers amplify regions of the SSU rRNA.

4. A method of claim 1 or 2, wherein said primers amplify regions of the LSU rRNA.

5. A method of claims 1 to 4, wherein at least one primer is specific for a variable region of SSU or LSU rRNA.

6. A method of claims 1 to 4, wherein at least one primer is specific for a helix region of SSU or LSU rRNA.

7. A method of claims 1 to 4, wherein said primers amplify a variable region of SSU or LSU rRNA.

8. A method of claim 7, wherein said primers are:
27F (5'-[6FAM] AGAGTTTGATCCTGGCTCAG-3') (SEQ ID NO: 37), and
338R' (5'-GCTGCCTCCCGTAGGAGT-3) (SEQ ID NO: 38).

9. A method of claims 1 to 8, wherein the disease is an inflammatory bowel disease, and further comprising performing PCO analysis to determine which reaction products indicate the presence of ulcerative colitis, pouchitis, or Crohn's disease.

10. A method of claims 1 to 9, further comprising sequencing said reaction products.

11. A method of claims 1 to 9, further comprising sequencing said reaction products and correlating said sequence with a microbe type.


SEQUENCE LISTING

<110> GEORGE MASON UNIVERSITY

<120> COMPOSITIONS AND METHODS FOR DIAGNOSING COLON DISORDERS

<130> GMU-001-WO

<140>
<141>

<150> 60/646,592
<151> 2005-01-26

<150> 60/673,771
<151> 2004-11-01

<160> 38

<170> PatentIn version 3.3

<210> 1
<211> 1489
<212> RNA
<213> Bacteroides distasonis

<220>
<221> modified_base
<222> (39)..(39)
<223> n is a, c, g, or u

<220>
<221> modified_base
<222> (99)..(99)
<223> n is a, c, g, or u

<220>
<221> modified_base
<222> (101)..(101)
<223> n is a, c, g, or u

<220>
<221> modified_base
<222> (108)..(108)
<223> n is a, c, g, or u

<220>
<221> modified_base
<222> (235)..(235)
<223> n is a, c, g, or u

<220>
<221> modified_base
<222> (482)..(482)
<223> n is a, c, g, or u

<220>
<221> modified_base
<222> (656)..(656)
<223> n is a, c, g, or u

<220>
<221> modified_base
<222> (932)..(932)
<223> n is a, c, g, or u

<220>
<221> modified_base

<222> (936)..(936)
<223> n is a, c, g, or u

<220>
<221> modified_base
<222> (1160)..(1160)
<223> n is a, c, g, or u

<400> 1
caauuuaaac aacgaagagu uugauccugg cucaggauna acgcuagcga caggcuuaac 60

acaugcaagu cgaggggcac gcgcgrguag caauaccgng ngcuggcnac cggcgcacgg 120

gugaguaacg cguaugcaac uugccuauca gagggggaua acccggcgaa agucggacua 180

auaccgcaug aagcagggau cccgcauggg aauauuugcu aaagauucau cgcunauaga 240

uaggcaugcg uuccauuagg caguuggcgg gguaacggcc caccaaaccg acgauggaua 300

gggguucuga gaggaagguc ccccacauug guacugagac acggaccaaa cuccuacggg 360

aggcagcagu gaggaauauu ggucaauggc cgagaggcug aaccagccaa gucgcgugag 420

ggaugaaggu ucuauggauc guaaaccucu uuuauaaggg aauaaagugc gggacguguc 480

cnguuuugua uguaccuuau gaauaaggau cggcuaacuc cgugccagca gccgcgguaa 540

uacggaggau ccgagcguua uccggauuua uuggguuuaa agggugcgua ggcggccuuu 600

uaagucagcg gugaaagucu guggcucaac cauagaauug ccguugaaac uggggngcuu 660

gaguauguuu gaggcaggcg gaaugcgugg uguagcggug aaaugcauag auaucacgca 720

gaaccccgau ugcgaaggca gccugccaag ccauuacuga cgcugaugca cgaaagcgug 780

gggaucaaac aggauuagau acccugguag uccacgcagu aaacgaugau cacuagcugu 840

uugcgauaca cuguaagcgg cacagcgaaa gcguuaagug auccaccugg ggaguacgcc 900

ggcaacggug aaacucaaag gaauugacgg gngccngcac aagcggagga acaugugguu 960

uaauucgaug auacgcgagg aaccuuaccc ggguuugaac gcauucggac cgagguggaa 1020

acaccuuuuc uagcaauagc cguuugcgag gugcugcaug guugucguca gcucgugccg 1080

ugaggugucg gcuuaagugc cauaacgagc gcaacccuug ccacuaguua cuaacagguu 1140

aggcugagga cucugguggn acugccagcg uaagcugcga ggaaggcggg gaugacguca 1200

aaucagcacg gcccuuacau ccggggcgac acacguguua caauggcgug gacaaaggga 1260

ggccaccugg cgacagggag cgaaucccca aaccacgucu caguucggau cggagucugc 1320

aacccgacuc cgugaagcug gauucgcuag uaaucgcgca ucagccaugg cgcggugaau 1380

acguucccgg gccuuguaca caccgcccgu caagccaugg gagccggggg uaccugaagu 1440

ccguaaccga aaggaucggc cuaggguaaa acuggugacu ggggcuaag 1489

<210> 2
<211> 1533
<212> RNA
<213> Bacteroides fragilis

<400> 2
   uuacaacgaa gaguuugauc cuggcucagg augaacgcua gcuacaggcu uaacacaugc 60
   aagucgaggg gcaucaggaa gaaagcuugc uuucuuugcu ggcgaccggc gcacggguga 120
   guaacacgua uccaaccugc ccuuuacucg gggauagccu uucgaaagaa agauuaauac 180
   ccgauagcau aaugauuccg caugguuuca uuauuaaagg auuccgguaa aggaugggga 240
   ugcguuccau uagguuguug gugagguaac ggcucaccaa gccuucgaug gauagggguu 300
   cugagaggaa ggucccccac auuggaacug agacacgguc caaacuccua cgggaggcag 360
   cagugaggaa uauuggucaa ugggcgcuag ccugaaccag ccaaguagcg ugaaggauga 420
   aggcucuaug ggucguaaac uucuuuuaua uaagaauaaa gugcaguaug uauacuguuu 480
   uguauguauu auaugaauaa ggaucggcua acuccgugcc agcagccgcg guaauacgga 540
   ggauccgagc guuauccgga uuuauugggu uuaaagggag cguaggugga cugguaaguc 600
   aguugugaaa guuugcggcu caaccguaaa auugcagcug auacugucag ucuugaguac 660
   aguagaggug ggcggaauuc gugguguagc ggugaaaugc uuagauauca cgaagaacuc 720
   cgauugcgaa ggcagcucac uggacugcaa cugacacuga ugcucgaaag uguggguauc 780
   aaacaggauu agauacccug guaguccaca caguaaacga ugaauacucg cuguuugcga 840
   uauacaguaa gcggccaagc gaaagcauua aguauuccac cuggggagua cgccggcaac 900
   ggugaaacuc aaaggaauug acgggggccc gcacaagcgg aggaacaugu gguuuaauuc 960
   gaugauacgc gaggaaccuu acccgggcuu aaauugcagu ggaaugaugu ggaaacaugu 1020
   cagugagcaa ucaccgcugu gaaggugcug caugguuguc gucagcucgu gccgugaggu 1080
   gucggcuuaa gugccauaac gagcgcaacc cuuaucuuua guuacuaaca gguuaugcug 1140
   aggacucuag agagacugcc gucguaagau gugaggaagg uggggaugac gucaaaucag 1200
   cacggcccuu acguccgggg cuacacacgu guuacaaugg gggguacaga aggcagcuag 1260
   cgggugaccg uaugcuaauc ccaaaauccu cucucaguuc ggaucgaagu cugcaacccg 1320
   acuucgugaa gcuggauucg cuaguaaucg cgcaucagcc acggcgcggu gaauacguuc 1380
   ccgggccuug uacacaccgc ccgucaagcc augggagccg gggguaccug aaguacguaa 1440
   ccgcaaggau cguccuaggg uaaaacuggu gacuggggcu aagucguaac aagguagccg 1500
   uaccggaagg ugcggcugga acaccuccuu ucu 1533

<210> 3
<211> 1468
<212> RNA
<213> Bacteroides ovatus


<220>
<221> modified_base
<222> (204)..(207)
<223> n is a, c, g, or u

<220>
<221> modified_base
<222> (339)..(339)
<223> n is a, c, g, or u

<220>

<221> modified_base
<222> (439)..(439)
<223> n is a, c, g, or u

<220>
<221> modified_base
<222> (530)..(530)
<223> n is a, c, g, or u

<220>
<221> modified_base
<222> (544)..(544)
<223> n is a, c, g, or u

<220>
<221> modified_base
<222> (700)..(700)
<223> n is a, c, g, or u

<220>
<221> modified_base
<222> (739)..(739)
<223> n is a, c, g, or u

<220>
<221> modified_base
<222> (788)..(788)
<223> n is a, c, g, or u

<220>
<221> modified_base
<222> (953)..(953)
<223> n is a, c, g, or u

<220>
<221> modified_base
<222> (962)..(962)
<223> n is a, c, g, or u

<220>
<221> modified_base
<222> (988)..(988)
<223> n is a, c, g, or u

<220>
<221> modified_base
<222> (1031)..(1031)
<223> n is a, c, g, or u

<220>
<221> modified_base
<222> (1084)..(1084)
<223> n is a, c, g, or u

<400> 3
augaagaguu ugauccuggc ucaggaugaa cgcuagcuac aggcuuaaca caugcaaguc 60

gaggggcagc auuuuaguuu gcuugcaaac ugaagauggc gaccggcgca cgggugagua 120

acacguaucc aaccugccga uaacuccggg auagccuuuc gaaagaaaga uuaauaccgg 180

aurgyauayg aacaucgcau gaunnnnuua uuaaagaauu ucgguuaucg auggggaugc 240

guuccauuag uuuguuggcg ggguaacggc ccaccaagac uacgauggau agggguucug 300

agaggaaggu cccccacauu ggaacugaga cacgguccna acuccuacgg gaggcagcag 360

ugaggaauau uggucaaugg gcgagagccu gaaccagcca aguagcguga aggaugaagg 420

cucuaugggu cguaaacunc uuuuauaugg gaauaaagug uuccacgugu ggaauuuugu 480
auguaccaua ugaauaagga ucggcuaacu ccgugccagc agccgcggun auacggagga 540
uccnagcguu auccggauuu auuggguuua aagggagcgu agguggauug uuaagucagu 600
ugugaaaguu ugcggcucaa ccguaaaauu gcaguugaaa cuggcagucu ugaguacagu 660
agaggugggc ggaauucgug guguagcggu gaaaugcuun gauaucacga agaacuccga 720
uugcgaaggc agcucacung acuguuacug acacugaugc ucgaaagugu ggguaucaaa 780
caggauunga uacccuggua guccacacag uaaacgauga auacucgcug uuugcgauau 840
acaguaagcg gccaagcgaa agcauuaagu auuccaccug gggaguacgc cggcaacggu 900
gaaacucaaa ggaauugacg ggggcccgca caagcggagg aacauguggu uunauucgau 960
gnuacgcgag gaaccuuacc cgggcuunaa uugcawcwga auauauwgga aacwruauag 1020
ccgyaaggca nuugugaagg ugcugcaugg uugucgucag cucgugccgu gaggugucgg 1080
cuunagugcc auaacgagcg caacccuuau cuuuaguuac uaacagguua ugcugaggac 1140
ucuagagaga cugccgucgu aagaugugag gaaggugggg augacgucaa aucagcacgg 1200
cccuuacguc cggggcuaca cacguguuac aauggggggu acagaaggcr gcuaccuggy 1260
gacaggaugc uaaucccaaa aaccucucuc aguucggauc gaagucugca acccgacuuc 1320
gugaagcugg auucgcuagu aaucgcgcau cagccauggc gcggugaaua cguucccggg 1380
ccuuguacac accgcccguc aagccaugaa agccgggggu accugaagua cguaaccgca 1440
aggagcgucc uaggguaaaa cugguaau 1468

<210> 4
<211> 1524
<212> RNA
<213> Bacteroides vulgatus

<220>
<221> modified_base
<222> (35)..(35)
<223> n is a, c, g, or u

<220>
<221> modified_base
<222> (97)..(97)
<223> n is a, c, g, or u

<220>
<221> modified_base
<222> (99)..(99)
<223> n is a, c, g, or u

<220>
<221> modified_base
<222> (395)..(395)
<223> n is a, c, g, or u

<220>
<221> modified_base
<222> (481)..(481)
<223> n is a, c, g, or u

<220>
<221> modified_base
<222> (544)..(544)
<223> n is a, c, g, or u

<220>
<221> modified_base
<222> (744)..(744)
<223> n is a, c, g, or u

<220>
<221> modified_base
<222> (932)..(932)
<223> n is a, c, g, or u

<220>
<221> modified_base
<222> (1204)..(1204)
<223> n is a, c, g, or u

<220>
<221> modified_base
<222> (1255)..(1255)
<223> n is a, c, g, or u

<220>
<221> modified_base
<222> (1499)..(1499)
<223> n is a, c, g, or u

<400> 4
uauuacaaug aagaguuuga uccuggcuca ggaunaacgc uagcuacagg cuuaacacau 60

gcaagucgag gggcagcaug gucuuagcuu gcuaagncna uggcgaccgg cgcacgggug 120

aguaacacgu auccaaccug ccgucuacuc uuggacagcc uucugaaagg aagauuaaua 180

caagauggca ucaugagucc gcauguucac augauuaaag guauuccggu agacgauggg 240

gaugcguucc auuagauagu aggcggggua acggcccacc uagucuucga uggauagggg 300

uucugagagg aagguccccc acauuggaac ugagacacgg uccaaacucc uacgggaggc 360

agcagugagg aauauugguc aaugggcgag agccngaacc agccaaguag cgugaaggau 420

gacugcccua uggguuguaa acuucuuuua uaaaggaaua aagucgggua uggauacccg 480

nuugcaugua cuuuaugaau aaggaucggc uaacuccgug ccagcagccg cgguaauacg 540

gagnauccga gcguuauccg gauuuauugg guuuaaaggg agcguagaug gauguuuaag 600

ucaguuguga aaguuugcgg cucaaccgua aaauugcagu ugauacugga uaucuugagu 660

gcaguugagg caggcggaau ucguggugua gcggugaaau gcuuagauau cacgaagaac 720

uccgauugcg aaggcagccu gcunagcugc aacugacauu gaggcucgaa agugugggua 780

ucaaacagga uuagauaccc ugguagucca cacgguaaac gaugaauacu cgcuguuugc 840

gauauacugc aagcggccaa gcgaaagcgu uaaguauucc accuggggag uacgccggca 900

acggugaaac ucaaaggaau ugacgggggc cngcacaagc ggaggaacau gugguuuaau 960

ucgaugauac gcgaggaacc uuacccgggc uuaaauugca gaugaauuac ggugaaagcc 1020

guaagccgca aggcaucugu gaaggugcug caugguuguc gucagcucgu gccgugaggu 1080

gucggcuuaa gugccauaac gagcgcaacc cuuguuguca guuacuaaca gguuaugcug 1140

aggacucuga caagacugcc aucguaagau gugaggaagg uggggaugac gucaaaucag 1200

cacngcccuu acguccgggg cuacacacgu guuacaaugg gggguacaga gggcngcuac 1260

cacgcgagug gaugccaauc cccaaaaccu cucucaguuc ggacuggagu cugcaacccg 1320

acuccacgaa gcuggauucg cuaguaaucg cgcaucagcc acggcgcggu gaauacguuc 1380

ccgggccuug uacacaccgc ccgucaaguc augggagccg gggguaccug aagugcguaa 1440

ccgcgaggag cgcccuaggg uaaaacuggu gacuggggcu aagucguaac aagguagcng 1500

uaccggaagg aacaccuccu uucu 1524

<210> 5
<211> 1173
<212> RNA
<213> Propionibacterium acnes

<400> 5
uuggagaguu ugauccuggc ucaggacgaa cgcuggcggc gugcuuaaca caugcaaguc 60

gaacggaaag gcccugcuuu uguggggugc ucgaguggcg aacgggugag uaacacguga 120

guaaccugcc cuugacuuug ggauaacuuc aggaaacugg ggcuaauacc ggauaggagc 180

uccugcugca uggugggggu uggaaaguuu cggcgguugg ggauggacuc gcggcuuauc 240

agcuuguugg ugggguagug gcuuaccaag gcuuugacgg guagccggcc ugagagggug 300

accggccaca uugggacuga gauacggccc agacuccuac gggaggcagc aguggggaau 360

auugcacaau gggcggaagc cugaugcagc aacgccgcgu gcgggaugac ggccuucggg 420

uuguaaaccg cuuucgccug ugacgaagcg ugagugacgg uaauggguaa agaagcaccc 480

gcuaacuacg ugccagcagc cgcggugaua cguagggugc caacguuguc cggauuuauu 540

gggcguaaag ggcucguagg ugguugaucg cgucggaagu guaaucuugg ggcuuaaccc 600

ugagcgugcu uucgauacgg guugacuuga ggaagguagg ggagaaugga auuccuggug 660

gagcggugga augcgcagau aucaggagga acaccagugg cgaaggcggu ucucugggcc 720

uuuccugacg cugaggagcg aaagcguggg gagcgaacag gcuuagauac ccugguaguc 780

cacgcuguaa acggugggua cuaggugugg gguccauucc acggguuccg ugccguagcu 840

aacgcuuuaa guaccccgcc uggggaguac ggccgcaagg cuaaaacuca aaggaauuga 900

cggggccccg cacaagcggc ggagcaugcg gauuaauucg augcaacgcg uagaaccuua 960

ccuggguuug acauggaucg ggagugcuca gagaugggug ugccucuuuu ggggucgguu 1020

cacagguggu gcauggcugu cgucagcucg ugucgugaga uguuggguua agucccgcaa 1080

cgagcgcaac ccuuguucuc uguugccagc acguuauggu ggggacucag uggagaccgc 1140

cggggucaac ucggaggaag guggggauga cgu 1173

<210> 6
<211> 1517
<212> RNA
<213> Clostridium nexile

<220>
<221> modified_base
<222> (184)..(185)
<223> n is a, c, g, or u

<220>
<221> modified_base
<222> (341)..(341)
<223> n is a, c, g, or u

<220>
<221> modified_base
<222> (430)..(430)
<223> n is a, c, g, or u

<400> 6
gagauuugau ccuggcucag gaugaacgcu ggccggccgu gcuuacacau gcagucgaac 60

gaagcgcuua aacuggauuu cuucggauug aaguuuuugc ugacugagug gcggacgggu 120

gaguaacgcg uggguaaccu gccucauaca gggggauaac aguuagaaau gacugcuaau 180

accnnauaag cgcacagugc ugcauggcac aguguaaaaa cuccgguggu augagaugga 240

cccgcgucug auuagcuagu ugguggggua acggccuacc aaggcgacga ucaguagccg 300

gccugagagg gugaacggcc acauugggac ugagacacgg nccaaacucc uacgggaggc 360

agcagugggg aauauugcac aaugggggaa acccugaugc agcgacgccg cgugagcgaa 420

gaaguauuun gguauguaaa gcucuaucag cagggaagaa aaugacggua ccugacuaag 480

aagcaccggc uaaauacgug ccagcagccg cgguaauacg uaggugcaag cguuauccgg 540

auuuacuggg uguaaaggga gcguagacgg uuguguaagu cugaugugaa agcccggggc 600

ucaaccccgg acugcauugg aaacuaugua acuagagugu cggagaggua agcggaauuc 660

cuaguguagc ggugaaaugc guagauauua ggaggaacac caguggcgaa ggcggcuuac 720

uggacgauca cugacguuga ggcucgaaag cguggggagc aaacaggauu agauacccug 780

guaguccacg ccguaaacga ugacuacuag gugucgggga gcaaagcucu ucggugccgc 840

agcaaacgca auaaguaguc caccugggga guacguucgc aagaaugaaa cucaaaggaa 900

uugacgggga cccgcacaag cguggagcau gugguuuaau ucgagcaacg cgaagaccuu 960

accuggucuu gacaucccgg ugaccggucc aguaauggga ccuuuccuuc gggacacggu 1020

gacagguggu gcaugguugu cgucagcucg ugucgugaga uguuggguua agucccgcaa 1080

cgagcgcaac cccuaucuuc aguagccagc auuuaaggug ggcacucugg agagacugcc 1140

agggauaacc uggaggaagg uggggaugac gucaaaucau caugccccuu augaccaggg 1200

cuacacacgu gcuacaaugg cguaaacaaa gggaagcgaa ccugugaggg gaagcaaauc 1260

ucaaaaauaa cgucucaguu cggauuguag ucugcaacuc gacuacauga agcuggaauc 1320

gcuaguaauc gcgaaucagc augucgcggu gaauacguuc ccgggucuug uacacaccgc 1380

ccgucacacc augggaguca guaacgcccg aagucaguga cccaaccgua aggagggagc 1440

ugccgaaggu gggaccgaua acugggguga agucguaaca agguagccgu aucggaaggu 1500

gcggcuggau caccucc                                                    1517

<210> 7
<211> 1480
<212> RNA
<213> Eubacterium formicigenerans

<220>
<221> modified_base
<222> (1)..(1)
<223> n is a, c, g, or u

<220>
<221> modified_base
<222> (124)..(126)
<223> n is a, c, g, or u

<220>
<221> modified_base
<222> (221)..(223)
<223> n is a, c, g, or u

<220>
<221> modified_base
<222> (288)..(288)
<223> n is a, c, g, or u

<220>
<221> modified_base
<222> (350)..(351)
<223> n is a, c, g, or u

<220>
<221> modified_base
<222> (518)..(518)
<223> n is a, c, g, or u

<220>
<221> modified_base
<222> (535)..(536)
<223> n is a, c, g, or u

<220>
<221> modified_base
<222> (726)..(726)
<223> n is a, c, g, or u

<220>
<221> modified_base
<222> (919)..(919)
<223> n is a, c, g, or u

<220>
<221> modified_base
<222> (922)..(922)
<223> n is a, c, g, or u

<220>
<221> modified_base
<222> (956)..(957)
<223> n is a, c, g, or u

<220>
<221> modified_base
<222> (1185)..(1186)
<223> n is a, c, g, or u

<400> 7
nuuaaacgag aguuugaucc uggcucagga ugaacgcugg cggcgugcuu aacacaugca 60

agucgagcga agcacauaag uuugauucuu cggaugaaga cuuuugugac ugagcggcgg 120

acgnnngagu aacgcguggg uaaccugccu cauacagggg gauaacagyu agaaauggcu 180

gcuaauaccg cauaagacca caguacugca ugguacagug nnnaaaacuc cggugguaug 240

agauggaccc gcgucugauu agguaguugg ugagguaacg gcccaccnag ccgacgauca 300

guagccgacc ugagagggug accggccaca uugggacuga gacacggccn ngacuccuac 360

gggaggcagc aguggggaau auugcacaau gggcgaaagc cugaugcagc gacgccgcgu 420

gaaggaugaa guauuucggu auguaaacuu cuaucagcag ggaagaaaau gacgguaccu 480

gacuaagaag ccccggcuaa cuacgugcca gcagccgngg uaauacguag ggggnnagcg 540

uuauccggau uuacugggug uaaagggagc guagacggcu gugcaagucu gaagugaaag 600

gcaugggcuc aaccugugga cugcuuugga aacugugcag cuagaguguc ggagagguaa 660

guggaauucc uaguguagcg gugaaaugcg uagauauuag gaggaacacc aguggcgaag 720

gcggcnuacu ggacgaugac ugacguugag gcucgaaagc guggggagca aacaggauua 780

gauacccugg uaguccacgc cguaaacgau gacugcuagg ugucggguag caaagcuauu 840

cggugccgca gcuaacgcaa uaagcagucc accuggggag uacguucgca agaaugaaac 900

ucaaaggaau ugacggggnc cngcacaagc gguggagcau gugguuuaau ucgaannaac 960

gcgaagaacc uuaccugauc uugacauccc gaugaccgcu ucguaaugga agyuuuucuu 1020

cggaacaucg gugacaggug gugcaugguu gucgucagcu cgugucguga gauguugggu 1080

uaagucccgc aacgagcgca acccuuaucu ucaguagcca gcauuuagga ugggcacucu 1140

ggagagacug ccagggauaa ccuggaggaa gguggggaug acgunnaauc aucaugcccc 1200

uuaugaccag ggcuacacac gugcuacaau ggcguaaaca gagggaggca gagccgcgag 1260

gccgagcaaa ucucaaaaau aacgucucag uucggauugu agucugcaac ucgacuacau 1320

gaagcuggaa ucgcuaguaa ucgcagauca gaaugcugcg gugaauacgu ucccgggucu 1380

uguacacacc gcccgucaca ccaugggagu caguaacgcc cgaagucagu gacccaaccg 1440

aaaggaggga gcugccgaag gugggaccga uaacuggggu 1480

<210> 8
<211> 1423
<212> DNA
<213> Ruminococcus gnavus


<220>
<221> modified_base
<222> (1148)..(1148)
<223> n is a, c, g, or t

<220>
<221> modified_base
<222> (1188)..(1188)
<223> n is a, c, g, or t

<220>
<221> modified_base
<222> (1257)..(1257)
<223> n is a, c, g, or t

<400> 8
cctggctcag gatgaacgct ggcggcgtgc ttaacacatg caagtcgagc gaagcacctt 60

gacggatttc ttcggattga agccttggtg actgagcggc ggacgggtga gtaacgcgtg 120

ggtaacctgc ctcatacagg gggataacag ttggaaacgg ctgctaatac cgcataagcg 180

cacagtaccg catggtacgg tgtgaaaaac tccggtggta tgagatggac ccgcgtctga 240

ttaggtagtt ggtggggtaa cggcctacca agccgacgat cagtagccga cctgagaggg 300

tgaccggcca cattgggact gagacacggc ccaaactcct acgggaggca gcagtgggga 360

atattgcaca atgggggaaa ccctgatgca gcgacgccgc gtgagcgatg aagtatttcg 420

gtatgtaaag ctctatcagc agggaagaaa atgacggtac ctgactaaga agccccggct 480

aactacgtgc cagcagccgc ggtaatacgt agggggcaag cgttatccgg atttactggg 540

tgtaaaggga gcgtagacgg catggcaagc cagatgtgaa agcccggggc tcaaccccgg 600

gactgcattt ggaactgtca ggctagagtg tcggagagga aagcggaatt cctagtgtag 660

cggtgaaatg cgtagatatt aggaggaaca ccagtggcga aggcggcttt ctggacgatg 720

actgacgttg aggctcgaaa gcgtggggag caaacaggat tagataccct ggtagtccac 780

gccgtaaacg atgaatacta ggtgtcgggt ggaaaagcca ttcggtgccg cagcaaacgc 840

aataagtatt ccacctgggg agtacgttcg caagaatgaa actcaaagga attgacgggg 900

acccgcacaa gcggtggagc atgtggttta attcgaagca acgcgaagaa ccttacctgg 960

tcttgacatc cctctgaccg ctctttaatc ggagctttcc ttcgggacag aggagacagg 1020

tggtgcatgg ttgtcgtcag ctcgtgtcgt gagatgttgg gttaagtccc gcaacgagcg 1080

caacccctat ctttagtagc cagcattttg gatgggcact ctagagagac tvccagggat 1140

aacctggngg aaggtgggga tgacgtcaaa tcatcatgcc ccttatgncc agggctacac 1200

acgtgctaca atggcgtaaa caaagggaag cgagcccgcg agggggagca aatcccnaaa 1260

ataacgtctc agttcggatt gtagtctgca actcgactac atgaagctgg aatcgctagt 1320

aatcgcgaat cagaatgtcg cggtgaatac gttcccgggt cttgtacaca ccscccgtca 1380

caccatggga gtmagtaacg cccgaagtca gtgacccaac cgc 1423

<210> 9
<211> 1418
<212> DNA
<213> Ruminococcus torques

<220>
<221> modified_base
<222> (724)..(724)
<223> n is a, c, g, or t

<220>

<221> modified_base
<222> (771)..(771)
<223> n is a, c, g, or t

<220>
<221> modified_base
<222> (776)..(776)
<223> n is a, c, g, or t

<220>
<221> modified_base
<222> (807)..(807)
<223> n is a, c, g, or t

<400> 9
ctcaggatga acgctggcgg cgtgcctaac acatgcaagt cgagcgaagc actttgctta 60

gattcttcgg atgaagagga ttgtgactga gcggcggacg ggtgagtaac gcgtgggtaa 120

cctgcctcat acagggggat aacagttaga aatgactgct aataccgcat aagaccacag 180

caccgcatgg tgcgggggta aaaactccgg tggtatgaga tggacccgcg tctgattagc 240

tagttggtaa ggtaacggct taccaaggcg acgatcagta gccgacctga gagggtgacc 300

ggccacattg ggactgagac acggcccaaa ctcctacggg aggcagcagt ggggaatatt 360

gcacaatggg ggaaaccctg atgcagcgac gccgcgtgag cgaagaagta tttcggtatg 420

taaagctcta tcagcaggga agaaaatgac ggtacctgac taagaagcac cggctaaata 480

cgtgccagca gccgcggtaa tacgtatggt gcaagcgtta tccggattta ctgggtgtaa 540

agggagcgta gacggatggg caagtctgat gtgaaaaccc ggggctcaac cccgggactg 600

cattggaaac tgttcatcta gagtgctgga gaggtaagtg gaattcctag tgtagcggtg 660

aaatgcgtag atattaggag gaacaccagt ggcgaaggcg gcttactgga cagtaactga 720

cgtngaggct cgaaagcgtg gggagcacac aggattagat accctggtag nccacnccgt 780

aaacgatgac tactaggtgt cgggtgncaa agccattcgg tgccgcagca aacgcaataa 840

gtagtccacc tggggagtac gttcgcaaga atgaaactca aaggaattga cggggacccg 900

cacaagcggt ggagcatgtg gtttaattcg aagcaacgcg aagaacctta cctgctcttg 960

acatcccgct gaccggacgg taatgcgtcc ttcccttcgg ggcagcggag acaggtggtg 1020

catggttgtc gtcagctcgt gtcgtgagat gttgggttaa gtcccgcaac gagcgcaacc 1080

cctatcttta gtagccagcg gccaggccgg gcactctaga gagactgccg gggataaccc 1140

ggaggaaggt ggggatgacg tcaaatcatc atgcccctta tgagcagggc tacacacgtg 1200

ctacaatggc gtaaacaaag ggaagcgaga ccgcgaggtg gagcaaatcc caaaaataac 1260

gtctcagttc ggattgtagt ctgcaactcg actacatgaa gctggaatcg ctagtaatcg 1320

cgaatcagaa tgtcgcggtg aatacgttcc cgggtcttgt acacaccgcc cgtcacacca 1380

tgggagtcag taacgcccga agtcagtgac ccaaccgt 1418

<210> 10
<211> 1458
<212> RNA
<213> Clostridium sp.

<400> 10
gaugaacgcu ggcggcgugc uuaacacaug caagucgagc gaagcgauuc uaawgaaguu 60

uucggaygga auuuraauug acugagcggc ggacggguga guaacgcgug gguaaccugc 120

cucauacagg gggauaacag uuggaaacgg cugcuaauac cgcauaagca cacagugccg 180

caugguacgg ugugaaaaac uccgguggua ugagauggac ccgcgucuga uuagguaguu 240

ggugagguaa cggcccacca agccgacgau caguagccga ccugagaggg ugaccggcca 300

cauugggacu gagacacggc ccaaacuccu acgggaggca gcagugggga auauuggaca 360

augggggaaa cccugaucca gcgacgccgc gugagugaag aaguauuucg guauguaaag 420

cucuaucagc agggaagaaa augacgguac cugacuaaga agccccggcu aacuacgugc 480

cagcagccgc gguaauacgu agggggcaag cguuauccgg auuuacuggg uguaaaggga 540

gcguagacgg caaugcaagu cuggagugaa agcccggggc ucaaccccgg gacugcuuug 600

gaaacugugu ugcuagagug caggagaggu aaguggaauu ccuaguguag cggugaaaug 660

cguagauauu aggaggaaca ccaguggcga aggcggcuua cuggacugua acugacguug 720

aggcucgaaa gcguggggag caaacaggau uagauacccu gguaguccac gccguaaacg 780

augaauacua gguguugggg agcaaagcuc uucggugccg ccgcuaacgc aauaaguauu 840

ccaccugggg aguacguucg caagaaugaa acucaaagga auugacgggg acccgcacaa 900

gcgguggagc augugguuua auucgaagca acgcgaagaa ccuuaccaag ucuugacauc 960

ggaaugaccg guccguaacg gggccuuccc uacggggcau uccagacagg uggugcaugg 1020

uugucgucag cucgugucgu gagauguugg guuaaguccc gcaacgagcg caacccuuau 1080

ccuuaguagc cagcauguag uggugggcac ucuggggaga cugccaggga uaaccuggag 1140

gaaggugggg augacgucaa aucaucaugc cccuuaugau uugggcuaca cacgugcuac 1200

aauggcguaa acaaagggaa gcaaaggagc gaucuuaagc aaaccccaaa aauaacgucu 1260

caguucggau uguagucugc aacucgacua caugaagcug gaaucgcuag uaaucgcgga 1320

ucagaaugcc gcggugaaua cguucccggg ucuuguacac accgcccguc acaccauggg 1380

aguugguaac gcccgaaguc agugacccam ccsymaggag ggagcugccg aaggcgggac 1440

urauaacugg ggugaaug 1458

<210> 11
<211> 728
<212> RNA
<213> Moraxella cuniculi

<400> 11
aggcuuaaca caugcaaguc gaacgaaguu agggagcuug cuccugauac uuaguggcgg 60

acgggugagu aaugcuuagg aaucugccua guaguggggg auaacuaucc gaaaggauag 120

cuaauaccgc auacgaccua cgggugaaag ggggcguaag cucucgcuau uagaugagcc 180

uaagucggau uagcuaguug gugggguaaa ggccuaccaa ggcgacgauc uguagcuggu 240

cugagaggau gaucagccac acugggacug agacacggcc cagacuccua cgggaggcag 300

caguggggaa uauuggacaa ugggcgaaag ccugauccag ccaugcccgc gugugugaag 360
aaggccuuuu gguuguaaag cacuuuaagu ggggaggaaa agcuaauagc uaauaccuau 420
uagcccugac guuacccaca gaauaagcac cggcuaacuc ugugccagca gccgcgguaa 480
uacagagggu gcaagcguua aucggaauua cugggcguaa agcgcgcgua ggugguuacu 540
uaagucagau gugaaagccc cgggcuuaac cugggaacug caucugauac uggguaacua 600
gaguagguga gagggaagua gaauuccagg uguagcggug aaaugcguag agaucuggag 660
gaauaccgau ggcgaaggca gcuuccuggc aucauacuga cacugaggug cgaaagcgug 720
gguagcaa 728

<210> 12
<211> 1519
<212> RNA
<213> Moraxella lacunata

<400> 12
agaguuugau cauggcucag auugaacgcu ggcggcaggc uuaacacaug caagucgaac 60
gaugaagucu agcuugcuag acggauuagu ggcgaacggg ugaguaaugc uuaggaaucu 120
gccuauuagu gggggauaac guagggaaac uuacgcuaau accgcauacg ucuuacgaga 180
gaaagggggc uuuuagcucu cgcuaauaga ugagccuaag ucggauuagc uaguuggugg 240
gguaaaggcc uaccaaggcg acgaucugua gcuggucuga gaggaugauc agccacacug 300
ggacugagac acggcccaga cuccuacggg aggcagcagu ggggaauauu ggacaauggg 360
cgaaagccug auccagccau gccgcgugug ugaagaaggc cuuuugguug uaaagcacuu 420
uaagugggga ggaaaagcuu gugguuaaua ccuacaagcc cugacguuac ccacagaaua 480
agcaccggcu aacucugugc cagcagccgc gguaauacag agggugcaag cguuaaucgg 540
aauuacuggg cguaaagcga gcguaggugg ucauuuaagu cagaugugaa agccccgggc 600
uuaaccuggg aacugcaucu gauacugggu gacuagagua ggugagaggg aaguagaauu 660
ccagguguag cggugaaaug cguagagauc uggaggaaua ccgauggcga aggcagcuuc 720
cuggcaucau acugacacug agguucgaaa gcguggguag caaacaggau uagauacccu 780
gguaguccac gccguaaacg augucuacca gucguugggu cucuugaaga cuuagugacg 840
caguuaacgc aauaaguaga ccgccugggg aguacggccg caagguuaaa acucaaauga 900
auugacgggg cccgcacaag cgguggagca ugugguuuaa uucgaugcaa cgcgaagaac 960
cuuaccuggu cuugacauag ugagaauccu gcagagaugc gggagugcuu cgggaauuca 1020
cauacaggug cugcauggcu gucgucagcc cgugucguga gauguugggu uaagucccgc 1080
aacgagcgca acccuuuucc uuaguuacca gcgauuuaag ucgggaacuc uaaggauacu 1140
gccagugaca aacuggagga aggcgggacg acgucaaguc aucauggccc uuacgaccag 1200
ggcuacacac gugcuacaau gguugguaca aaggguugcu acacagcgau gugaugcuaa 1260
ucucaaaaag ccaaucguag uccggauugg agucugcaac ucgacuccau gaagucggaa 1320

ucgcuaguaa ucgcagauca gaaugcugcg gugaauacgu ucccgggccu uguacacacc 1380

gcccgucaca ccaugggagu ugaucucacc agaagugguu agccuaacgc aagagggcga 1440

ucaccacggu ggggucgaug acugggguga agucguaaca agguagccgu aggggaacug 1500

cgguuggauc accuccuua 1519

<210> 13
<211> 1448
<212> RNA
<213> Moraxella osloensis

<400> 13
cuggcggcag gcuuaacaca ugcaagucga acgaugacuc ucuagcuugc uagagaugau 60

uaguggcgga cgggugagua acauuuagga aucugccuag uaguggggga uagcucgggg 120

aaacucgaau uaauaccgca uacgaccuac gggugaaagg gggcgcaagc ucuugcuauu 180

agaugagccu aaaucagauu agcuaguugg ugggguaaag gcccaccaag gcgacgaucu 240

guaacugguc ugagaggaug aucagucaca ccggaacuga gacacggucc ggacuccuac 300

gggaggcagc aguggggaau auuggacaau gggggcaacc cugauccagc caugccgcgu 360

gugugaagaa ggccuuuugg uuguaaagca cuuuaagcag ggaggagagg cuaaugguua 420

auacccauua gauuagacgu uaccugcaga auaagcaccg gcuaacucug ugccagcagc 480

cgcgguaaua cagagggugc gagcguuaau cggaauuacu gggcguaaag cgaguguagg 540

uggcucauua agucacaugu gaaauccccg ggcuuaaccu gggaacugca ugugauacug 600

guggugcuag aauaugugag agggaaguag aauuccaggu guagcgguga aaugcguaga 660

gaucuggagg aauaccgaug gcgaaggcag cuuccuggca uaauauugac acugagauuc 720

gaaagcgugg guagcaaaca ggauuagaua cccugguagu ccacgccgua aacgaugucu 780

acuagccguu gggguccuug agacuuuagu ggcgcaguua acgcgauaag uagaccgccu 840

ggggaguacg gccgcaaggu uaaaacucaa augaauugac gggggcccgc acaagcggug 900

gagcaugugg uuuaauucga ugcaacgcga agaaccuuac cuggucuuga cauagugaga 960

aucucucaga gaugagagag ugccuucggg aacucacaua caggugcugc auggcugucg 1020

ucagcucgug ucgugagaug uuggguuaag ucccgcaacg agcgcaaccc uuuuccuuau 1080

uugccagcgg guuaagccgg gaacuuuaag gauacugcca gugacaaacu ggaggaaggc 1140

ggggacgacg ucaagucauc auggcccuua cgaccagggc uacacacgug cuacaauggu 1200

agguacagag gguugcuaca cagcgaugug augcuaaucu caaaaagccu aucguagucc 1260

ggauuggagu cugcaacucg acuccaugaa gucggaaucg cuaguaaucg cggaucagaa 1320

ugccgcggug aauacguucc cgggccuugu acacaccgcc cgucacacca ugggagucua 1380

uugcaccaga aguagguagc cuaacgaaag agggcgcuua ccacggugug gucgaugacu 1440

ggggugaa 1448

<210> 14
<211> 452

<212> RNA
<213> Acinetobacter junii

<400> 14
caugcaaguc gagcggagau gaggugcuug caccuuaucu uagcggcgga cgggugagua 60

augcuuagga aucugccuau uaguggggga caacauuccg aaaggaaugc uaauaccgca 120

uacguccuac gggagaaagc aggggaucuu cggaccuugc gcuaauagau gagccuaagu 180

cggauuagcu aguugguggg guaaaggccu accaaggcga cgaucuguag cgggucugag 240

aggaugaucc gccacacugg gacugagaca cggcccagac uccuacggga ggcagcagug 300

gggaauauug gacaaugggg ggaacccuga uccagccaug ccgcgugugu gaagaaggcc 360

uuaugguugu aaagcacuuu aagcgaggag gaggcuacug agacuaauac ucuuggauag 420

uggacguuac ucgcagaaua agcaccggcu aa 452

<210> 15
<211> 1488
<212> RNA
<213> Comamonas sp.

<400> 15
auugaacgcu ggcggcaugc cuuacacaug caagucgaac gguaacaggu cuucggaugc 60

ugacgagugg cgaacgggug aguaauacau cggaacgugc ccgauygugg gggauaacga 120

ggcgaaagcu uugcuaauac cgcauacgau cuacggauga aagcggggga ucuucggacc 180

ucgcgcggac ggagcggccg auggcagauu agguaguugg ugggauaaaa gcuuaccaag 240

ccgacgaucu guagcugguc ugagaggaug aucagccaca cugggacuga gacacggccc 300

agacuccuac gggaggcagc aguggggaau uuuggacaau gggggaaacc cugauccagc 360

caugccgcgu gcaggaugaa ggccuucggg uuguaaacug cuuuuguacg gaacgaaaag 420

gucucuucua auaaaggggg cccaugacgg uaccguaaga auaagcaccg gcuaacuacg 480

ugccagcagc cgcgguaaua cguagggugc aagcguuaau cggaauuacu gggcguaaag 540

cgugcgcagg cgguuaugua agacagaugu gaaauccccg ggcucaaccu gggaacugca 600

uuugugacug cauggcuuga gugcggcaga gggggaugga auuccgcgug uagcagugaa 660

augcguagau augcggagga acaccgaugg cgaaggcaau ccccugggcc ugcacugacg 720

cucaugcacg aaagcguggg gagcaaacag gauuagauac ccugguaguc cacgcccuaa 780

acgaugucaa cugguuguug ggaauuuguu uucucaguaa cgaagcuaac gcgugaaguu 840

gaccgccugg ggaguacggc cgcaagguug aaacucaaag gaauugacgg ggacccgcac 900

aagcggugga ugaugugguu uaauucgaug caacgcgaaa aaccuuaccc accuuugaca 960

uggcaggaag uccacagaga ugaggaugug cucgaaagag aaccugcaca caggugcugc 1020

auggcugucg ucagcucgug ucgugagaug uuggguuaag ucccgcaacg agcgcaaccc 1080

uugucauuag uugcuacauu uaguugggca cucuaaugag acugccggug acaaaccgga 1140

ggaagguggg gaugacguca aguccucaug gcccuuauag guggggcuac acacgucaua 1200

caauggcugg uacaaagggu ugccaacccg cgagggggag cuaaucccau aaagccaguc 1260

guaguccgga ucgcagucug caacucgacu gcgugaaguc ggaaucgcua guaaucgugg 1320

aucagaaugu cacggugaau acguucccgg gucuuguaca caccgcccgu cacaccaugg 1380

gagcgggucu cgccagaagu agguagccua accgcaagga gggcgcuuac cacggcgggg 1440

uucgugacug gggugaaguc guaacaaggu agccguaucg gaaggugc 1488

<210> 16
<211> 1520
<212> RNA
<213> Comamonas terrigena

<220>
<221> modified_base
<222> (623)..(623)
<223> n is a, c, g, or u

<220>
<221> modified_base
<222> (630)..(630)
<223> n is a, c, g, or u

<400> 16
aguuugaucc uggcucagau ugaacgcugg cggcaugcuu uacacaugca agucgaacgg 60

cagcacggac uucggucugg uggcgagugg cgaacgggug aguaauacau cggaacgugc 120

ccaguugugg gggauaacua cucgaaagag uagcuaauac cgcaugagua cugagguuga 180

aagcagggga ucgcaagacc uugcgcaacu ggagcggccg auggcagauu agguaguugg 240

ugggauaaaa gcuuaccaag ccgacgaucu guagcugguc ugagaggacg accagccaca 300

cugggacuga gacacggccc agacuccuac gggaggcagc aguggggaau uuuggacaau 360

gggcgaaagc cugauccagc aaugccgcgu gcaggaugaa ggccuucggg uuguaaacug 420

cuuuuguacg gaacgaaaag cuucggguua auaccuggag ucaugacggu acccuaagaa 480

uaagcaccgg cuaacuacgu gccagcagcc gcgguaauac guagggugca agcguuaauc 540

ggaauuacug ggcguaaagc gugcgcaggc ggucuuguaa gacagaggug aaauccccgg 600

gcucaaccug ggaacugccu uunugacugn aaggcuggag ugcggcagag ggggauggaa 660

uuccgcgugu agcagugaaa ugcguagaua ugcggaggaa caccgauggc gaaggcaauc 720

cccugggccu gcacugacgc ucaugcacga aagcgugggg agcaaacagg auuagauacc 780

cugguagucc acggccuaaa cgaugucaac ugguuguugg gucuuaacug acucaguaac 840

gaagcuaacg cgugaaguug accgccuggg gaguacggcc gcaagguuga aacucaaagg 900

aauugacggg gacccgcaca agcgguggau gaugugguuu aauuugaugc aacgcgaaaa 960

accuuaccca ccuuugacau guacggaauc cuuuagagau agaggagugc ucgaaagaga 1020

gccguaacac aggugcugca uggcugucgu cagcucgugu cgugagaugu uggguuaagu 1080

cccgcaacga gcgcaacccu ugccauuagu ugcuacgaaa gggcacucua augggacuuc 1140

cggugacaaa ccggaggaag guggggauga cgucaagucc ucauggcccu uauagguggg 1200

gcuacacacg ucauacaaug gcugguacaa aggguugcca acccgcgagg gggagcuaau 1260

cccauaaagc cagucguagu ccgguucgca gucugcaacu cgacugcgug aagucggaau 1320

cgcuaguaau cguggaucag aaugucacgg ugaauacguu cccgggucuu guacacaccg 1380

cccgucacac caugggagcg ggucucgcca gaaguaggua gccuaaccgu aaggagggcg 1440

cuuaccacgg cgggguucgu gacuggggug aagucguaac aagguagccg uaucggaagg 1500

ugcggcugga ucaccuccuu 1520

<210> 17
<211> 1476
<212> RNA
<213> Bergeyella zoohelcum

<220>
<221> modified_base
<222> (138)..(138)
<223> n is a, c, g, or u

<220>
<221> modified_base
<222> (141)..(143)
<223> n is a, c, g, or u

<220>
<221> modified_base
<222> (183)..(183)
<223> n is a, c, g, or u

<220>
<221> modified_base
<222> (278)..(278)
<223> n is a, c, g, or u

<220>
<221> modified_base
<222> (285)..(285)
<223> n is a, c, g, or u

<220>
<221> modified_base
<222> (317)..(318)
<223> n is a, c, g, or u

<220>
<221> modified_base
<222> (341)..(342)
<223> n is a, c, g, or u

<220>
<221> modified_base
<222> (344)..(344)
<223> n is a, c, g, or u

<220>
<221> modified_base
<222> (528)..(528)
<223> n is a, c, g, or u

<220>
<221> modified_base
<222> (532)..(533)
<223> n is a, c, g, or u

<220>
<221> modified_base
<222> (542)..(542)
<223> n is a, c, g, or u

<220>
<221> modified_base
<222> (547)..(547)
<223> n is a, c, g, or u

<220>
<221> modified_base
<222> (737)..(737)
<223> n is a, c, g, or u

<220>
<221> modified_base
<222> (766)..(767)
<223> n is a, c, g, or u

<220>
<221> modified_base
<222> (965)..(966)
<223> n is a, c, g, or u

<220>
<221> modified_base
<222> (1022)..(1022)
<223> n is a, c, g, or u

<220>
<221> modified_base
<222> (1085)..(1086)
<223> n is a, c, g, or u

<220>
<221> modified_base
<222> (1096)..(1096)
<223> n is a, c, g, or u

<220>
<221> modified_base
<222> (1205)..(1205)
<223> n is a, c, g, or u

<220>
<221> modified_base
<222> (1288)..(1289)
<223> n is a, c, g, or u

<220>
<221> modified_base
<222> (1384)..(1384)
<223> n is a, c, g, or u

<220>
<221> modified_base
<222> (1406)..(1406)
<223> n is a, c, g, or u

<400> 17
   auacaaugga gaguuugauc cuggcucagg augaacgcua gcgggaggcc uaacacaugc      60
   aagccgagcg ggauuuguug guuagcuugc uaacuaacaa ugagagcggc guacgggugc     120
   guaacacgug ugcaaccngc nnnuaucugg gggauagccu uucgaaagga agauuaauac     180
   ccnauaauau auugauuggc aucaguuaau auugaaagcu ccggcggaua gagaugggca     240
   cgcguaagau uagcuaguug gugagguaac ggcucacnaa ggcuncgauc uuuagggggc 300
   cugagagggu gaucccnnac acugguacug agacacggac nngncuccua cgggaggcag 360
   cagugaggaa uauuggacaa ugggugagag ccugauccag ccaucccgcg ugaaggacua 420
   aggaccuaug guuuguaaac uucuuuuaua cagggauaaa ccuacucucg ugaggguagc 480
   ugaagguacu guaugaauaa gcaccggcua acuccgugcc agcagccncg gnnauacgga 540
   gngugcnagc guuauccgga uuuauugggu uuaaaggguc cguaggcggg ucgauaaguc 600
   aguggugaaa gccugcagcu uaacuguaga acugccguug auacugucgg ucuugagugu 660
   auuugaggua gcuggaauga guaguguagc ggugaaaugc auagauauua cucagaacac 720
   caauugcgaa ggcaggnuac caaguuacaa cugacgcuga uggacnnaag cguggggagc 780
   gaacaggauu agauacccug guaguccacg cuguaaacga ugcuaacucg uuuuuggggu 840
   auuauacuuc agagaccaag cgaaagugau aaguuagcca ccuggggagu acgaacgcaa 900
   guuugaaacu caaaggaauu gacgggggcc cgcacaagcg guggauuaug ugguuuaauu 960
   cgaunnuacg cgaggaaccu uaccaagacu uaaaugggaa uugacagcug uagaaauacg 1020
   gnuuucuucg gacaauuuuc aaggugcugc augguugucg ucagcucgug ccgugaggug 1080
   uuagnnuaag uccugnaacg agcgcaaccc cugucacuag uugccaucau uaaguugggg 1140
   acucuaguga gacugccuac gcaaguagag aggaaggugg ggaugacguc aaaucaucac 1200
   ggccnuuacg ucuugggcca cacacguaau acaauggccg guacagaggg cagcuacacu 1260
   gcgaagugau gcgaaucucg aaagccgnnc ucaguucgga uuggagucug caacucgacu 1320
   cuaugaagcu ggaaucgcua guaaucgcgc aucagccaug gcgcggugaa uacguucccg 1380
   ggcnuuguac acaccgcccg ucaagncaug gaagucuggg guaccugaag ucggugaccg 1440
   uuaaaggagc ugccuagggu aaaacaggua acuagg 1476

<210> 18
<211> 395
<212> RNA
<213> Chryseobacterium balustinum str. SBR1044

<220>
<221> modified_base
<222> (343)..(343)
<223> n is a, c, g, or u

<400> 18
uagcgggagg gcuaacacau gcaagccgag cgguauuguu ucuucggaaa ugagagagcg 60

gcguacgggu gcggaacacg ugugcaaccu gccuuuaucu gggggauagc cuuucgaaag 120

gaagauuaau acuccauaac auauugaacg gcaucguuug auauugaaag cuccggcgga 180

uagagauggg cacgcgcaag auuagcuagu uggugaggua acggcucacc aaggcgauga 240

ucuuuagggg ggcugagagg gugauccccc acacugguac ugagacacgg accagacucc 300

uacgggaggc agcagugagg aauauugguc aaugggugca agncugaacc agccaucccg 360

cgugaaggac gacugcccua uggguuguaa acuuc                                395

<210> 19
<211> 395
<212> RNA
<213> Chryseobacterium balustinum str. SBR2024

<220>
<221> modified_base
<222> (11)..(11)
<223> n is a, c, g, or u

<220>
<221> modified_base
<222> (14)..(14)
<223> n is a, c, g, or u

<220>
<221> modified_base
<222> (17)..(18)
<223> n is a, c, g, or u

<220>
<221> modified_base
<222> (310)..(310)
<223> n is a, c, g, or u

<400> 19
uagcgggagg ncunacnnau gcaagccgag cgguauuguu ucuucggaaa ugagagagcg      60

gcguacgggu gcggaacacg ugugcaaccu gccuuuaucu gggggauagc cuuucgaaag     120

gaagauuaau acuccauaac auauugauug gcaucaauua auauugaaag cuccggcgga     180

uagagauggg cacgcgcaag auuagcuagu uggugaggua acggcucacc aaggcgauga     240

ucuuuagggg ggcugagagg gugauccccc acacugguac ugagacacgg accagacucc     300

uacgggaggn agcagugagg aauauugguc aaugggugca agccugaacc agccaucccg     360

cgugaaggac gacugcccua uggguuguaa acuuc                                395

<210> 20
<211> 1435
<212> RNA
<213> Clostridium paraputrificum

<400> 20
cgaacgcugg cggcgugccu aacacaugca agucgagcga ugaaguuccu ucgggaacgg      60

auuagcggcg gacgggugag uaacacgugg gcaaccugcc uuauagaggg gaauagccuu     120

ccgaaaggaa gauuaauacc gcauaagauu guagcuucgc augaaguagc aauuaaagga     180

gcaauccgcu auaagauggg cccgcggcgc auuagcuagu uggugaggua acggcucacc     240

aaggcgacga ugcguagccg accugagagg gugaucggcc acauugggac ugagacacgg     300

cccagacucc uacgggaggc agcagugggg aauauugcac aaugggggaa acccugaugc     360

agcaacgccg cgugagugau gacggccuuc ggguuguaaa gcucugucuu uggggacgau     420

aaugacggua cccaaggagg aagccacggc uaacuacgug ccagcagccg cgguaauacg     480

uagguggcaa gcguuguccg gauuuacugg gcguaaaggg agcguaggcg gauuuuuaag     540

ugggauguga aauacccggg cucaaccugg gugcugcauu ccaaacugga aaucuagagu 600

gcaggagggg aaaguggaau uccuagugua gcggugaaau gcguagagau uaggaagaac 660

accaguggcg aaggcgacuu ucuggacugu aacugacgcu gaggcucgaa agcgugggga 720

gcaaacagga uuagauaccc ugguagucca cgccguaaac gaugaauacu agguguaggg 780

guugucauga ccucugugcc gccgcuaacg cauuaaguau uccgccuggg gaguacgguc 840

gcaagauuaa aacucaaagg aauugacggg ggcccgcaca aguagcggag cauguggguu 900

aauucgaagc aacgcgaaga accuuaccua gacuugacau cuccugaauu accauguaau 960

gugggaaguc ccuucgggga caggaagaca gguggugcau gguugucguc agcucguguc 1020

gugagauguu ggguuaaguc ccgcaacgag cgcaacccuu auuguuaguu gcuaccauuu 1080

aguugagcac ucuagcgaga cugcccgggu uaaccgggag gaaggugggg augacgucaa 1140

aucaucaugc cccuuauguc uagggcuaca cacgugcuac aauggccggu acaacgagau 1200

gcaauaccgu gagguggagc aaaacuauaa aaccggucuc aguucggauu guaggcugaa 1260

acucgccuac augaagcugg aguuacuagu aaucgcgaau cagaaugucg cggugaauac 1320

guucccgggc cuuguacaca ccgcccguca caccaugaga guuggcaaua cccaaaguug 1380

gugaucuaac ccguaaggga ggaagccacc uaagguaggg ucagcgauug gggug 1435

<210> 21
<211> 1509
<212> RNA
<213> Enterococcus cecorum

<400> 21
   gacgaacgcu ggcggcgugc cuaauacaug caagucgaac gcauuuucuu ucaccguagc 60
   uugcuacacc ggaagaaaau gaguggcgaa cgggugagua acacgugggu aaccugccca 120
   ucagcggggg auaacacuug gaaacaggug cuaauaccgc auaauuccau uuaccgcaug 180
   guagauggau gaaaggcgcu uuugcgucac ugauggaugg acccgcggug cauuagcuag 240
   uugguggggu aacggccuac caaggcugcg augcauagcc gaccugagag ggugaucggc 300
   cacacuggga cugagacacg gcccagacuc cuacgggagg cagcaguagg gaaucuucgg 360
   caauggacgc aagucugacc gagcaacgcc gcgugaguga agaagguuuu cggaucguaa 420
   aacucuguug uuagagaaga acaaggauga gaguggaaag uucaucccuu gacgguaucu 480
   aaccagaaag ccacggcuaa cuacgugcca gcagccgcgg uaauacguag guggcaagcg 540
   uuguccggau uuauugggcg uaaagcgagc gcaggcgguc uuuuaagucu gaugugaaag 600
   cccccggcuu aaccggggag ggucauugga aacugggaga cuugagugca gaagaggaaa 660
   gcggaauucc auguguagcg gugaaaugcg uagauauaug gaggaacacc aguggcgaag 720
   gcggcuuucu ggucuguaac ugacgcugag gcucgaaagc guggggagca aacaggauua 780
   gauacccugg uaguccacgc cguaaacgau gagugcuaag uguuggaggg uuuccgcccu 840
   ucagugcugc agcaaacgca uuaagcacuc cgccugggga guacgaccgc aagguugaaa 900
   cucaaaggaa uugacgggga cccgcacaag cgguggagca ugugguuuaa uucgaagcaa 960
   cgcgaagaac cuuaccaggu cuugacaucc uuugaccauc cuagagauag gauuuucccu 1020
   ucggggacaa agugacaggu ggugcauggu ugucgucagc ucgugucgug agauguuggg 1080
   uuaagucccg caacgagcgc aacccuuauu guuaguugcc aucauucagu ugggcacucu 1140
   agcgagacug ccgcagacaa ugcggaggaa gguggggaug acgucaaauc aucaugcccc 1200
   uuaugaccug ggcuacacac gugcuacaau ggagaguaca acgagucgca aagccgcgag 1260
   gcuaagccaa ucucuuaaag cucuucucag uucggauugu aggcugcaac ucgccuacau 1320
   gaagccggaa ucgcuaguaa ucgcggauca gcacgccgcg gugaauacgu ucccgggucu 1380
   uguacacacc gcccgucaca ccacgagagu uuguaacacc caaagccggu gcgguaaccg 1440
   caaggagcca gccgucuaag gugggauaga ugauuggggu gaagucguaa caagguagcc 1500
   guaucggaa 1509

<210> 22
<211> 1493
<212> RNA
<213> Enterococcus columbae


<220>
<221> modified_base
<222> (33)..(33)
<223> n is a, c, g, or u

<220>
<221> modified_base
<222> (103)..(103)
<223> n is a, c, g, or u

<220>
<221> modified_base
<222> (158)..(158)
<223> n is a, c, g, or u

<220>
<221> modified_base
<222> (194)..(196)
<223> n is a, c, g, or u

<220>
<221> modified_base
<222> (209)..(209)
<223> n is a, c, g, or u

<220>
<221> modified_base
<222> (230)..(230)
<223> n is a, c, g, or u

<220>
<221> modified_base
<222> (254)..(254)
<223> n is a, c, g, or u

<220>
<221> modified_base
<222> (289)..(289)
<223> n is a, c, g, or u

<220>
<221> modified_base
<222> (316)..(316)
<223> n is a, c, g, or u

<220>
<221> modified_base
<222> (346)..(347)
<223> n is a, c, g, or u

<220>
<221> modified_base
<222> (367)..(367)
<223> n is a, c, g, or u

<220>
<221> modified_base
<222> (430)..(432)
<223> n is a, c, g, or u

<220>
<221> modified_base
<222> (441)..(441)
<223> n is a, c, g, or u

<220>
<221> modified_base
<222> (492)..(492)
<223> n is a, c, g, or u

<220>
<221> modified_base
<222> (555)..(556)
<223> n is a, c, g, or u

<220>
<221> modified_base
<222> (565)..(565)
<223> n is a, c, g, or u

<220>
<221> modified_base
<222> (613)..(613)
<223> n is a, c, g, or u

<220>
<221> modified_base
<222> (639)..(639)
<223> n is a, c, g, or u

<220>
<221> modified_base
<222> (783)..(783)
<223> n is a, c, g, or u

<220>
<221> modified_base
<222> (788)..(788)
<223> n is a, c, g, or u

<220>
<221> modified_base
<222> (792)..(792)
<223> n is a, c, g, or u

<220>
<221> modified_base

<222> (811)..(811)
<223> n is a, c, g, or u

<220>
<221> modified_base
<222> (871)..(871)
<223> n is a, c, g, or u

<220>
<221> modified_base
<222> (898)..(898)
<223> n is a, c, g, or u

<220>
<221> modified_base
<222> (965)..(965)
<223> n is a, c, g, or u

<220>
<221> modified_base
<222> (979)..(979)
<223> n is a, c, g, or u

<220>
<221> modified_base
<222> (1013)..(1013)
<223> n is a, c, g, or u

<220>
<221> modified_base
<222> (1035)..(1037)
<223> n is a, c, g, or u

<220>
<221> modified_base
<222> (1063)..(1063)
<223> n is a, c, g, or u

<220>
<221> modified_base
<222> (1068)..(1068)
<223> n is a, c, g, or u

<220>
<221> modified_base
<222> (1071)..(1071)
<223> n is a, c, g, or u

<220>
<221> modified_base
<222> (1102)..(1102)
<223> n is a, c, g, or u

<220>
<221> modified_base
<222> (1127)..(1128)
<223> n is a, c, g, or u

<220>
<221> modified_base
<222> (1228)..(1228)
<223> n is a, c, g, or u

<220>
<221> modified_base
<222> (1337)..(1337)
<223> n is a, c, g, or u

<220>
<221> modified_base
<222> (1399)..(1399)
<223> n is a, c, g, or u

<220>
<221> modified_base
<222> (1401)..(1401)
<223> n is a, c, g, or u

<220>
<221> modified_base
<222> (1424)..(1424)
<223> n is a, c, g, or u

<220>
<221> modified_base
<222> (1462)..(1462)
<223> n is a, c, g, or u

<400> 22
ugagaguuug auccuggcuc aggacgaacg cungcggcgu gccuaauaca ugcaagucga 60

acgcacuuuc uuucaccgua gcuugcuaca ccgaaaguaa gunaguggcg aacgggugag 120

uuacacgugg guaaccugcc caucagcggg ggauaacncu uggaaacagg ugcuaauacc 180

gcauaauauu acunnncgca ugagaaguna uugaaaggcg caacugcgun acugauggau 240

ggacccgcgg ugcnuuagcu aguuggugag guaacggccu accaaggcna cgaugcauag 300

ccgaccugag agggunaucg gccacacugg gacugagaca cggccnnaac uccuacggga 360

ggcagcngua gggaaucuuc ggcaauggac gcaagucuga ccgagcaacg ccgcgugagu 420

gaagaaggun nncggaucgu naaacucugu uguuagagaa gaacagggau gagaguggaa 480

aguucauccc ungacgguau cuaaccagaa agccacggcu aacuacgugc cagcagccgc 540

gguaauacgu aggunncaag cguunuccgg auuuauuggg cguaaagcga gcgcaggcgg 600

ucuuuuaagu cunaugugaa agcccacggc uuaaccgung agggucauug gaaacuggga 660

gacuugagug cagaagagga aagcggaauu ccauguguag cggugaaaug cguagauaua 720

ugcaggaaca ccaguggcga aggcggcuuu cuggucugua acugacgcug aggcucgaaa 780

gcnugggnag gnaacaggau uagauacccu nguaguccac gccguaaacg augagugcua 840

aguguuggag gguuuccgcc cuucagugcu ncagcaaacg cauuaagcac uccgccungg 900

gaguacgacc gcaagguuga aacucaaagg aauugacggg gaccgcacaa gcgguggagc 960

augunguuua auucgaagna acgcgaagaa ccuuaccagg ucuugacauc cunugaccau 1020

ccuagagaua ggacnnnccu ucggggacaa agugacaggu ggngcaungu ngucgucagc 1080

ucgugucgug agauguuggg unaagucccg caacgagcgc aacccunnuu guuaguugcc 1140

aucauuuagu ugggcacucu agcgagacug ccgcagacaa ugcggaggaa gguggggaug 1200

acgucaaauc aucaugcccc uuaugacnug ggcuacacac gugcuacaau ggagaguaca 1260

acgaguugcg aagucgugag gcuaagcuaa ucucuuaaag cucuucucag uucggauugu 1320

aggcugcaac ucgccuncau gaagccggaa ucgcuaguaa ucgcggauca gcacgccgcg 1380

gugaauacgu ucccgggunu nguacacacc gcccgucaca ccangagagu uuguaacacc 1440

cgaagccggu ggggtaaccg cnaggagcca gccgucuaag gugggauaga uga 1493

<210> 23
<211> 1535
<212> RNA
<213> Enterococcus hirae

<400> 23
ccuggcucag gacgaacgcu ggcggcgugc cuaauacaug caagucgaac gcuucuuuuu 60

ccaccggagc uugcuccacc ggaaaaagag gaguggcgaa cgggugagua acacgugggu 120

aaccugccca ucagaagggg auaacacuug gaaacaggug cuaauaccgu auaacaaucg 180

aaaccgcaug guuuugauuu gaaaggcgcu uucgggugug gcugauggau ggacccgcgg 240

ugcauuagcu aguuggugag guaacggcuc accaaggcga cgaugcauag ccgaccugag 300

agggugaucg gccacauugg gacugagaca cggcccaaac uccuacggga ggcagcagua 360

gggaaucuuc ggcaauggac gaaagucuga ccgagcaacg ccgcgugagu gaagaagguu 420

uucggaucgu aaaacucugu uguuagagaa gaacaaggau gagaguaacu guucaucccu 480

ugacgguauc uaaccagaaa gccacggcua acuacgugcc agcagccgcg guaauacgua 540

gguggcaagc guuguccgga uuuauugggc guaaagcgag cgcaggcggu uucuuaaguc 600

ugaugugaaa gcccccggcu caaccgggga gggucauugg aaacugggag acuugagugc 660

agaagaggag aguggaauuc cauguguagc ggugaaaugc guagauauau ggaggaacac 720

caguggcgaa ggcggcucuc uggucuguaa cugacgcuga ggcucgaaag cguggggagc 780

aaacaggauu agauacccug guaguccacg ccguaaacga ugagugcuaa guguuggagg 840

guuuccgccc uucagugcug cagcuaacgc auuaagcacu ccgccugggg aguacgaccg 900

caagguugaa acucaaagga auugacgggg gcccgcacaa gcgguggagc augugguuua 960

auucgaagca acgcgaagaa ccuuaccagg ucuugacauc cuuugaccac ucuagagaua 1020

gagcuucccc uucgggggca aagugacagg uggugcaugg uugucgucag cucgugucgu 1080

gagauguugg guuaaguccc gcaacgagcg caacccuuau uguuaguugc caucauuuag 1140

uugggcacuc uagcaagacu gccggugaca aaccggagga agguggggau gacgucaaau 1200

caucaugccc cuuaugaccu gggcuacaca cgugcuacaa ugggaaguac aacgagucgc 1260

aaagucgcga ggcuaagcua aucucuuaaa gcuucucuca guucggauug uaggcugcaa 1320

cucgccuaca ugaagccgga aucgcuagua aucgcggauc agcacgccgc ggugaauacg 1380

uucccgggcc uuguacacac cgcccgucac accacgagag uuuguaacac ccgaagucgg 1440

ugagguaacc uuuuggagcc agccgccuaa ggugggauag augauugggg ugaagucgua 1500

acaagguagc cguaucggaa ggugcggcug gauca 1535

<210> 24
<211> 1435
<212> DNA
<213> Tetragenococcus halophilus

<220>
<221> modified_base
<222> (6)..(6)
<223> n is a, c, g, or t

<220>
<221> modified_base
<222> (53)..(53)
<223> n is a, c, g, or t

<220>
<221> modified_base
<222> (253)..(253)
<223> n is a, c, g, or t

<220>
<221> modified_base
<222> (298)..(298)
<223> n is a, c, g, or t

<220>
<221> modified_base
<222> (1094)..(1094)
<223> n is a, c, g, or t

<220>
<221> modified_base
<222> (1182)..(1182)
<223> n is a, c, g, or t

<220>
<221> modified_base
<222> (1426)..(1426)
<223> n is a, c, g, or t

<400> 24
aatacntgca agtcgaacgc tgcttaagaa gaaacttcgg tttttcttaa agnggagtgg 60

cggacgggtg agtaacacgt ggggaaccta tccatcagcg ggggataaca cttggaaaca 120

ggtgctaata ccgcatatgg ctttttttca cctgaaagaa agctcaaagg cgctttacag 180

cgtcactgat ggctggtccc gcggtgcatt agccagttgg tgaggtaacg gctcaccaaa 240

gcaacgatgc atngccgacc tgagagggtg atcggccaca ctgggactga gacacggncc 300

agactcctac gggaggcagc agtagggaat cttcggcaat ggacgcaagt ctgaccgagc 360

aacgccgcgt gagtgaagaa ggttttcgga tcgtaaagct ctgttgtcag caaagaacag 420

gagaaagagg aaatgctttt tctatgacgg tagctgacca gaaagccacg gctaactacg 480

tgccagcagc cgcggtaata cgtaggtggc aagcgttgtc cggatttatt gggcgtaaag 540

cgagcgcagg cggtgattta agtctgatgt gaaagccccc agctcaactg gggagggtca 600

ttggaaactg gatcacttga gtgcagaaga ggagagtgga attccatgtg tagcggtgaa 660

atgcgtagat atatggagga acaccagtgg cgaaggcggc tctctggtct gtaactgacg 720

ctgaggctcg aaagcgtggg tagcaaacag gattagatac cctggtagtc cacgccgtaa 780

acgatgagtg ctaagtgttg gagggtttcc gcccttcagt gctgcagtta acgcattaag 840

cactccgcct ggggagtacg accgcaaggt tgaaactcaa aggaattgac gggggcccgc 900

acaagcggtg gagcatgtgg tttaattcga agcaacgcga agaaccttac caggtcttga 960

catcctttga ccgccctaga gatagggttt ccccttcggg ggcaaagtga caggtggtgc 1020

atggttgtcg tcagctcgtg tcgtgagatg ttgggttaag tcccgtaacg agcgcaaccc 1080

ttattgttag ttgncagcat tgagttgggc actctagcaa gactgccggt gacaaaccgg 1140

aggaaggcgg ggatgacgtc aaatcatcat gccccttatg anctgggcta cacacgtgct 1200

acaatgggaa gtacaacgag caagccaagc cgcaaggcct agcgaatctc tgaaagcttc 1260

tctcagttcg gattgcaggc tgcaactcgc ctgcatgaag ccggaatcgc tagtaatcgc 1320

ggatcagcat gccgcggtga atccgttccc gggccttgta cacaccgccc gtcacaccac 1380

gagagtttgt aacacccaaa gtcggtgcgg caacccttcg gggagncagc cgcct 1435

<210> 25
<211> 1452
<212> RNA
<213> Escherichia coli

<220>
<221> modified_base
<222> (491)..(492)
<223> n is a, c, g, or u

<220>
<221> modified_base
<222> (567)..(567)
<223> n is a, c, g, or u

<220>
<221> modified_base
<222> (571)..(571)
<223> n is a, c, g, or u

<220>
<221> modified_base
<222> (852)..(853)
<223> n is a, c, g, or u

<400> 25
aguuugauca uggcucagau ugaacgcugg cggcaggccu aacacaugca agucgaacgg 60

uaacaggaac gagcuugcug cuuugcugac gaguggcgga cgggugagua augucuggga 120

aacugccuga uggaggggga uaacuacugg aaacgguagc uaauaccgca uaacgucgca 180

agaccaaaga gggggaccuu cgggccucuu gccaucggau gugcccagau gggauuagcu 240

aguagguggg guaaaggcuc accuaggcga cgaucccuag cuggucugag aggaugacca 300

gccacacugg aacugagaca cgguccagac uccuacggga ggcagcagug gggaauauug 360

cacaaugggc gcaagccuga ugcagccaug ccgcguguau gaagaaggcc uucggguugu 420

aaaguacuuu cagcggggag gaagggagua aaguuaauac cuuugcucau ugacguuacc 480

cgcagaagaa nnaccggcua acuccgugcc agcagccgcg guaauacgga gggugcaagc 540

guuaaucgga auuacugggc guaaagngca ngcaggcggu uuguuaaguc agaugugaaa 600

uccccgggcu caaccuggga acugcaucug auacuggcaa gcuugagucu cguagagggg 660

gguagaauuc cagguguagc ggugaaaugc guagagaucu ggaggaauac cgguggcgaa 720

ggcggccccc uggacgaaga cugacgcuca ggugcgaaag cguggggagc aaacaggauu 780

agauacccug guaguccacg ccguaaacga ugucgacuug gagguugugc ccuugaggcg 840

uggcuuccgg annuaacgcg uuaagucgac cgccugggga guacggccgc aagguuaaaa 900

cucaaaugaa uugacggggg ccgcacaagc gguggagcau gugguuuaau ucgaugcaac 960

gcgaagaacc uuaccugguc uugacaucca cggaaguuuu cagagaugag aaugugccuu 1020

cgggaaccgu gagacaggug cugcauggcu gucgucagcu cguguuguga aauguugggu 1080

uaagucccgc aacgagcgca acccuuaucc uuuguugcca gcgguccggc cgggaacuca 1140

aaggagacug ccagugauaa acuggaggaa gguggggaug acgucaaguc aucauggccc 1200

uuacgaccag ggcuacacac gugcuacaau ggcgcauaca aagagaagcg accucgcgag 1260

agcaagcgga ccucauaaag ugcgucguag uccggauugg agucugcaac ucgacuccau 1320

gaagucggaa ucgcuaguaa ucguggauca gaaugccacg gugaauacgu ucccgggccu 1380

uguacacacc gcccgucaca ccaugggagu ggguugcaaa agaaguaggu agcuuaaccu 1440

ucgggagggc gc 1452

<210> 26
<211> 1541
<212> RNA
<213> Salmonella bovis

<400> 26
aaauugaaga guuugaucau ggcucagauu gaacgcuggc ggcaggccua acacaugcaa 60

gucgaacggu aacaggaaga agcuugcucg cugcugacga guggcggacg ggugcguaau 120

gucugggaaa cugccugaug gagggggaua acuacuggaa acgguggcua aucccgcaua 180

acgucgcaag accaaagagg gggaccucca ggccucuucc caucggaugu gcccagaugg 240

gauuagcuag uuggugaggu aacggcucac caaggcgacg aucccuagcu ggucugagag 300

gaugaccagc cacacuggaa cugagacacg guccagacuc cuacgggagg cagcaguggg 360

gaauauugca cagugugcgc aagccugaug cagccaugcc gccuguauga agaaggccuu 420

cggguuguaa aguacuuuca gcggggagga agguguugug guuaauaacu gcagcaauug 480

acguuacccg cagaagaagc accggcuaac uccgugccag cagccgcggu aauacggagg 540

gugcaagcgu uaaucggaau uacugggcgu aaagcgcacg caggcgguuu guuaagucag 600

augugaaauc cccgggcuca accugggaac ugcaucugau acuggcaagc uugagucucg 660

uagagggggg uagaauucca gguguagcgg ugaaaugcgu agagaucugg aggaauaccg 720

guggcgaagg cggcccccug gacgaagacu gacgcucagg ugcgaaagcg uggggagcaa 780

acaggauuag auacccuggu aguccacgcc guaaacgaug ucuacuugga gguugugccc 840

uugaggcgug gcuuccggag cuaacgcguu aaguagaccg ccuggggagu acggccgcaa 900

gguuaaaacu caaaugaauu gacgggggcc cgcacaagcg guggagcaug ugguuuaauu 960

ccaugcaacu cuaagaaccu uaccugguca ugacauccac agaacuuucc agagaugaga 1020

cugugccuuc gggaacugug agacaggugc ugcauggcug ucgucagcuc guguugugaa 1080

auguuggguu aagucccgca acgagcgcaa cccuuauccu uuguugccag cgguccggcc 1140

gggaacucaa aggagacugc cagugauaaa cuggaggaag guggggauga cgucaaguca 1200

ucaugccccu uacgaccagg gcuacacacg ugcuacaaug gcgcauacaa agagaagcga 1260

ccucgcgaga gcaagcggac cucauaaagu gcgucguagu ccggauugga gucugcaacu 1320

cgacuccaug aagucggaau cgcuaguaau cguggaucag aaugccacgg ugaauacguu 1380

cccgggccuu guacacaccg cccgucacac caugggagug gguugcaaaa gaaguaggua 1440

gcuuaaccuu cgggagggcg cuuaccacuu ugugauucau gacuggggug aagucguaac 1500

aagguaaccg uaggggaacc ugcgguugga ucaccuccuu a 1541

<210> 27
<211> 1488
<212> RNA
<213> Shigella boydii

<400> 27
uggcucagau ugaacgcugg cggcaggccu aacacaugca agucgaacgg uaacaggaag 60

cagcuugcug uuucgcugac gaguggcgga cgggugagua augucuggga aacugccuga 120

uggaggggga uaacuacugg aaacgguagc uaauaccgca uaacgucgca agaccaaaga 180

gggggaccuu cgggccucuu gccaucggau gugcccagau gggauuagcu uguugguggg 240

guaacggcuc accaaggcga cgaucccuag cuggucugag aggaugacca gccacacugg 300

aacugagaca cgguccagac uccuacggga ggcagcagug gggaauauug cacaaugggc 360

gcaagccuga ugcagccaug ccgcguguau gaagaaggcc uucggguugu aaaguacuuu 420

cagcggggag gaagggagua aaguuaauac cuuugcucau ugacguuauc cgcagaagaa 480

gcaccggcua acuccgugcc agcagccgcg guaauacgga gggugcaagc guuaaucgga 540

auuacugggc guaaagcgca cgcaggcggu uuguuaaguc agaugugaaa uccccgggcu 600

caaccuggga acugcaucug auacuggcaa gcuugagucu cguagagggg gguagaauuc 660

cagguguagc ggugaaaugc guagagaucu ggaggaauac cgguggcgaa ggcggccccc 720

uggacgaaga cugacgcuca ggugcgaaag cguggggagc aaacaggauu agauacccug 780

guaguccacg ccguaaacga ugucgacuug gagguugugc ccuugaggcg uggcuuccgg 840

agcuaacgcg uuaagucgac cgccugggga guacggccgc aagguuaaaa cucaaaugaa 900

uugacggggg cccgcacaag cgguggagca ugugguuuaa uucgaugcaa cgcgaagaac 960

cuuaccuggu cuugacaucc acggaaguuu ucagagauga gaaugugccu ucgggaaccg 1020

ugagacaggu gcugcauggc ugucgucagc ucguguugug aaauguuggg uuaagucccg 1080

caacgagcgc aacccuuauc cuuuguugcc agcgguccgg ccgggaacuc aaaggagacu 1140

gccagugaua aacuggagga agguggggau gacgucaagu caucauggcc cuuacgacca 1200

gggcuacaca cgugcuacaa uggcgcauac aaagagaagc gaccucgcga gagcaagcgg 1260

accucauaaa gugcgucgua guccggauug gagucugcaa cucgacucca ugaagucgga 1320

aucgcuagua aucguggauc agaaugccac ggugaauacg uucccgggcc uuguacacac 1380

cgcccgucac accaugggag uggguugcaa aagaaguagg uagcuuaacc uucgggaggg 1440

cgcuuaccac uuugugauuc augacugggg ugaagucgua acaaggua 1488

<210> 28
<211> 1471
<212> RNA
<213> Shigella dysenteriae

<220>
<221> modified_base
<222> (1)..(2)
<223> n is a, c, g, or u

<220>
<221> modified_base
<222> (1470)..(1470)
<223> n is a, c, g, or u

<400> 28
nnauugaaga guuugaucau ggcucagauu gaacgcuggc ggcaggccua acacaugcaa 60

gucgaacggu aacaggaagc agcuugcugc uuugcugacg aguggcggac gggugaguaa 120

ugucugggaa acugccugau ggagggggau aacuacugga aacgguagcu aauaccgcau 180

aacgucgcaa gaccaaagag ggggaccuuc gggccucuug ccaucggaug ugcccagaug 240

ggauuagcua guaggugggg uaauggcuca ccuaggcgac gaucccuagc uggucugaga 300

ggaugaccag ccacacugga acugagacac gguccagacu ccuacgggag gcagcagugg 360

ggaauauugc acaaugggcg caagccugau gcagccaugc cgcguguaug aagaaggcuu 420

cggguuguaa aguacuuuca gcggggagga agggaguaaa guuaauaccu uugcucauug 480

acguuacccg cagaagaagc accggcuaac uccgugccag cagccgcggu aauacggagg 540

gugcaagcgu uaaucggaau uacugggcgu aaagcgcacg caggcgguuu guuaagucag 600

augugaaauc cccgggcuca accugggaac ugcaucugau acuggcaagc uugagucucg 660

uagagggggg uagaauucca gguguagcgg ugaaaugcgu agagaucugg aggaauaccg 720

guggcgaagg cggcccccug gacgaagacu gacgcucagg ugcgaaagcg uggggagcaa 780

acaggauuag auacccuggu aguccacgcc guaaacgaug ucgacuugga gguugugccc 840

uugaggcgug gcuuccggag cuaacgcguu aagucgaccg ccuggggagu acggccgcaa 900

gguuaaaacu caaaugaauu gacgggggcc gcacaagcgg uggagcaugu gguuuaauuc 960

gaugcaacgc gaagaaccuu accuggucuu gacauccacg gaaguuuuca gagaugagaa 1020

ugugccuucg ggaaccguga gacaggugcu gcauggcugu cgucagcucg uguugugaaa 1080

uguuggguua agucccgcaa cgagcgcaac ccuuauccuu uguugccagc gguccggccg 1140

ggaacucaaa ggagacugcc agugauaaac uggaggaagg uggggaugac gucaagucau 1200

cauggcccuu acgaccaggg cuacacacgu gcuacaaugg cgcauacaaa gagaagcgac 1260

cucgcgagag caagcggacc ucauaaagug cgucguaguc cggauuggag ucugcaacuc 1320

gacuccauga agucggaauc gcuaguaauc guggaucaga augccacggu gaauacguuc 1380

ccgggccuug uacacaccgc ccgucacacc augggagugg guugcaaaag aaguagguag 1440

cuuaacuucg ggagggcgcu uaccacuuun u 1471

<210> 29
<211> 1468
<212> RNA
<213> Shigella flexneri

<220>
<221> modified_base
<222> (1)..(2)
<223> n is a, c, g, or u

<220>
<221> modified_base
<222> (372)..(372)
<223> n is a, c, g, or u

<400> 29
nnauugaaga guuugaucau ggcucagauu gaacgcuggc ggcaggccua acacaugcaa 60

gucgaacggu aacaggaagc agcuugcugu uucgcugacg aguggcggac gggugaguaa 120

ugucugggaa acugccugau ggagggggau aacuacugga aacgguagcu aauaccgcau 180

aacgucgcaa gaccaaagag ggggaccuuc gggccucuug ccaucggaug ugcccagaug 240

ggauuagcua guaggugggg uaacggcuca ccuaggcgac gaucccuagc uggucugaga 300

ggaugaccag ccacacugga acugagacac gguccagacu ccuacgggag gcagcagugg 360

ggaauauugc anaaugggcg caagccugau gcagccaugc cgcguguaug aagaaggccu 420

ucggguugua aaguacuuuc agcggggagg aagggaguaa aguuaauacc uuugcucauu 480

gacguuaccc gcagaagaag caccggcuaa cuccgugcca gcagccgcgg uaauacggag 540

ggugcaagcg uuaaucggaa uuacugggcg uaaagcgcac gcaggcgguu uguuaaguca 600

gaugugaaau ccccgggcuc aaccugggaa cugcaucuga uacuggcaag cuugagucuc 660

guagaggggg guagaauucc agguguagcg gugaaaugcg uagagaucug gaggaauacc 720

gguggcgaag gcggcccccu ggacgaagac ucacgcucag gugcgaaagc guggggagca 780

aacaggauua gauacccugg uaguccacgc uguaaacgau gucgacuugg agguugugcc 840

cuugaggugu ggcuuccgga cguaacgcgu uaagucgacc gccuggggag uacggccgca 900

agguuaaaac ucaaaugaau ugacgggggc cgcacaagcg guggagcaug ugguuuaauu 960

cgaugcaacg cgaagaaccu uaccuggucu ugacauccac ggaaguuuuc agagaugaga 1020

augugccuuc gggaaccgug agacaggugc ugcauggcug ucgucagcuc guguugugaa 1080

auguuggguu aagucccgca acgagcgcaa cccuuauccu uuguugccag cgguccggcc 1140

gggaacucaa aggagacugc cagugauaaa cuggaggaag guggggauga cgucaaguca 1200

ucauggcccu uacgaccagg gcuacacacg ugcuacaaug gcgcauacaa agagaagcga 1260

ccucgcgaga gcaagcggac cucacaaagu gcgucguagu ccggauugga gucugcaacu 1320

cgacuccaug aagucggaau cgcuaguaau cguggaucag aaugccacgg ugaauacguu 1380
cccgggccuu guacacaccg cucgucacac caugggagug gguuguaaaa gaaguaggua 1440
gcuuaacuuc gggagggcgc uuaccacu 1468

<210> 30
<211> 1541
<212> RNA
<213> Streptococcus bovis

<400> 30
agaguuugau ccuggcucag gacgaacgcu ggcggcgugc cuaauacaug caaguagaac 60
gcugaagacu uuagcuugcu aaaguuggaa gaguugcgaa cgggugagua acgcguaggu 120
aaccugccua cuagcggggg auaacuauug gaaacgauag cuaauaccgc auaacagcau 180
uuaacacaug uuagaugcuu gaaaggagca auugcuucac uaguagaugg accugcguug 240
uauuagcuag uuggugaggu aacggcucac caaggcgacg auacauagcc gaccugagag 300
ggugaucggc cacacuggga cugagacacg gcccagacuc cuacgggagg cagcaguagg 360
gaaucuucgg caaugggggc aacccugacc gagcaacgcc gcgugaguga agaagguuuu 420
cggaucguaa agcucuguug uaagagaaga acguguguga gaguggaaag uucacacagu 480
gacgguaacu uaccagaaag ggacggcuaa cuacgugcca gcagccgcgg uaauacguag 540
gucccgagcg uuguccggau uuauugggcg uaaagcgagc gcaggcgguu uaauaagucu 600
gaaguuaaag gcaguggcuu aaccauuguu cgcuuuggaa acuguuagac uugagugcag 660
aaggggagag uggaauucca uguguagcgg ugaaaugcgu agauauaugg aggaacaccg 720
guggcgaaag cggcucucug gucuguaacu gacgcugagg cucgaaagcg uggggagcaa 780
acaggauuag auacccuggu aguccacgcc guaaacgaug agugcuaggu guuaggcccu 840
uuccggggcu uagugccgca gcuaacgcau uaagcacucc gccuggggag uacgaccgca 900
agguugaaac ucaaaggaau ugacgggggc ccgcacaagc gguggagcau gugguuuaau 960
ucgaagcaac gcgaagaacc uuaccagguc uugacauccc gaugcuauuc cuagagauag 1020
gaaguuucuu cggaacaucg gugacaggug gugcaugguu gucgucagcu cgugucguga 1080
gauguugggu uaagucccgc aacgagcgca accccuauug uuaguugcca ucauuaaguu 1140
gggcacucua gcgagacugc cgguaauaaa ccggaggaag guggggauga cgucaaauca 1200
ucaugccccu uaugaccugg gcuacacacg ugcuacaaug guugguacaa cgagucgcga 1260
gucggugacg gcaagcaaau cucuuaaagc caaucucagu ucggauugua ggcugcaacu 1320
cgccuacaug aagucggaau cgcuaguaau cgcggaucag cacgccgcgg ugaauacguu 1380
cccgggccuu guacacaccg cccgucacac cacgagaguu uguaacaccc gaagucggug 1440
agguaaccuu uuaggagcca gccgccuaag gugggauaga ugauuggggu gaagucguaa 1500
caagguagcc guaucggaag gugcggcugg aucaccuccu u 1541

<210> 31

<211> 1492
<212> RNA
<213> Streptococcus infantarius

<400> 31
gcucaggacu aacgcuggcg gcgugccuaa uacaugcaag uagaacgcug aaaacuuuag 60

cuugcuaaag uuugaagagu ugcgaacggg ugaguaacgc guagguaacc ugccuacuag 120

cgggggauaa cuauuggaaa cgauagcuaa uaccgcauaa cagcauuuaa cccauguuag 180

augcuugaaa ggagcaauug cuucacuagu agauggaccu gcguuguauu agcuaguugg 240

ugagguaacg gcucaccaag gcgacgauac auagccgacc ugagagggug aucggccaca 300

cugggacuga gacacggccc agacuccuac gggaggcagc aguagggaau cuucggcaau 360

gggggcaacc cugaccgagc aacgccgcgu gagugaagaa gguuuucgga ucguaaagcu 420

cuguuguaag agaagaaugu gugugagagu ggaaaguuca cacagugacg guaacuuacc 480

agaaagggac ggcuaacuac gugccagcag ccgcgguaau acguaggucc cgagcguugu 540

ccggauuuau ugggcguaaa gcgagcgcag gcgguuuaau aagucugaag uuaaaggcag 600

uggcuuaacc auuguucgcu uuggaaacug uuagacuuga gugcagaagg ggagagugga 660

auuccaugug uagcggugaa augcguagau auauggagga acaccggugg cgaaagcggc 720

ucucuggucu guaacugacg cugaggcucg aaagcguggg gagcaaacag gauuagauac 780

ccugguaguc cacgccguaa acgaugagug cuagguguua ggcccuuucc ggggcuuagu 840

gccgcagcua acgcauuaag cacuccgccu ggggaguacg accgcaaggu ugaaacucaa 900

aggaauugac gggggcccgc acaagcggug gagcaugugg uuuaauucga agcaacgcga 960

agaaccuuac caggucuuga caucccgaug cuauuccuag agauaggaag uuucuucgga 1020

acaucgguga cagguggugc augguugucg ucagcucgug ucgugagaug uuggguuaag 1080

ucccgcaacg agcgcaaccc cuauuguuag uugccaucau uaaguugggc acucuagcga 1140

gacugccggu aauaaaccgg aggaaggugg ggaugacguc aaaucaucau gccccuuaug 1200

accugggcua cacacgugcu acaaugguug guacmacgag ucgcgagucg gugacggcaa 1260

gcaaaucucu uaaagccaau cucaguucgg auuguaggcu gcaacucgcc uacaugaagu 1320

cggaaucgcu aguaaucgcg gaucagcacg ccgcggugaa uacguucccg ggccuuguac 1380

acaccgcccg ucacaccacg agaguuugua acacccgaag ucggugaggu aaccuuuuag 1440

gagccagccg ccuaaggugg gauagaugau uggggugaag ucguaacaag gu 1492

<210> 32
<211> 1487
<212> RNA
<213> Streptococcus salivarius

<220>
<221> modified_base
<222> (939)..(939)
<223> n is a, c, g, or u

<220>

<221> modified_base
<222> (1059)..(1059)
<223> n is a, c, g, or u

<400> 32
uuuaaugaga guuugauccu ggcucaggac gaacgcuggc ggcgugccua auacaugcaa      60

guagaacgcu gaagagagga gcuugcucuu cuuggaugag uugcgaacgg gugaguaacg     120

cguagguaac cugccuugua gcgggggaua acuauuggaa acgauagcua auaccgcaua     180

acaauggaug acccauguca uuuauuugaa aggggcaaau gcuccacuac aagauggacc     240

ugcguuguau uagcuaguag gugagguaac ggcucaccua ggcgacgaua cauagccgac     300

cugagagggu gaucggccac acugggacug agacacggcc cagacuccua cgggaggcag     360

caguagggaa ucuucggcaa ugggggcaac ccugaccgag caacgccgcg ugagugaaga     420

agguuuucgg aucguaaagc ucuguuguaa gucaagaacg agugugagag uggaaaguuc     480

acacugugac gguagcuuac cagaaaggga cggcuaacua cgugccagca gccgcgguaa     540

uacguagguc ccgagcguug uccggauuua uugggcguaa agcgagcgca ggcgguuuga     600

uaagucugaa guuaaaggcu guggcucaac cauaguucgc uuuggaaacu gucaaacuug     660

agugcagaag gggagagugg aauuccaugu guagcgguga aaugcguaga uauauggagg     720

aacaccggug gcgaaagcgg cucucugguc uguaacugac gcugaggcuc gaaagcgugg     780

ggagcgaaca ggauuagaua cccugguagu ccacgccgua aacgaugagu gcuagguguu     840

ggauccuuuc cgggauucag ugccgcagcu aacgcauuaa gcacuccgcc uggggaguac     900

gaccgcaagg uugaaacuca aaggaauuga cgggggccng cacaagcggu ggagcaugug     960

guuuaauucg aagcaacgcg aagaaccuua ccaggucuug acaucccgau gcuauuucua    1020

gagauagaaa guuacuucgg uacaucggug acagguggng caugguuguc gucagcucgu    1080

gucgugagau guuggguuaa gucccgcaac gagcgcaacc ccuauuguua guugccauca    1140

uucaguuggg cacucuagcg agacugccgg uaauaaaccg gaggaaggug gggaugacgu    1200

caaaucauca ugccccuuau gaccugggcu acacacgugc uacaaugguu gguacaacga    1260

guugcgaguc ggugacggca agcuaaucuc uuaaagccaa ucucaguucg gauuguaggc    1320

ugcaacucgc cuacaugaag ucggaaucgc uaguaaucgc ggaucagcac gccgcgguga    1380

auacguuccc gggccuugua cacaccgccc gucacaccac gagaguuugu aacacccgaa    1440

gucggugagg uaaccuuuug gagccagccg ccuaaggugg gauagau                  1487

<210> 33
<211> 1540
<212> RNA
<213> Streptococcus thermophilus

<220>
<221> modified_base
<222> (130)..(130)
<223> n is a, c, g, or u

<400> 33

agaguuugau ccuggcucag gacgaacggu ggcggcgugc cuaauacaug caaguagaac 60

gcugaagaga ggagcuugcu cuucuuggau gaguugcgaa cgggugagua acgcguaggu 120

aaccugccun guagcggggg auaacuauug gaaacgauag cuaauaccgc auaacaaugg 180

augacacaug ucauuuauuu gaaaggggca auugcuccac uacaagaugg accugcguug 240

uauuagcuag uaggugaggu aauggcuuac cuaggcgacg auacauagcc gaccugagag 300

ggugaucggc cacacuggga cugagacacg gcccagacuc cuacgggagg cagcaguagg 360

gaaucuucgg caaugggggc aacccugacc gagcaacgcc gcgugacuga agaagguuuu 420

cggaucguaa agcucuguug uaagucaaga acggguguga gaguggaaag uucacacagu 480

gacgguagcu uaccagaaag ggacggcuaa cuacgugcca gcagccgcgg uaauacguag 540

gucccgagcg uuguccggau uuauugggcg uaaagcgagc gcaggcgguu ugauaagucu 600

gaaguuaaag gcuguggcuc aaccauaguu cgcuuuggaa acugucaaac uugagugcag 660

aaggggagag uggaauucca uguguagcgg ugaaaugcgu agauauaugg aggaacaccg 720

guggcgaaag cggcucucug gucuguaacu gacgcugagg cucgaaagcg uggggagcga 780

acaggauuag auacccuggu aguccacgcc guaaacgaug agugcuaggu guuggauccu 840

uuccgggauu cagugccgca gcuaacgcau uaagcacucc gccuggggag uacgaccgga 900

agguugaaac ucaaaggaau ugacggggcc cgcacaagcg guggagcaug ugguuuaauu 960

cgaagcaacg cgaagaaccu uaccaccucu ugacaucccg augcuauuuc uagagauaga 1020

aaguuacuuu gguacaucgg ugacaggugg ugcaugguug ucgucagcuc gugucgugag 1080

auguuggguu aagucccgca acgagcgcaa ccccuauugu uaguugccau cauucaguug 1140

ggcacucuag cgagacugcc gguaauaaac cggaggaagg uggggaugac gucaaaucau 1200

caugccccuu augaccuggg cuacacacgu gcuacaaugg uugguacaac gaguugcgag 1260

ucggugacgg cgagcuaauc ucuuaaagcc aaucucaguu cggauuguag gcugcaacuc 1320

gccuacauga agucggaauc gcuaguaauc gcggaucagc acgccgcggu gaauacguuc 1380

ccgggccuug uacacaccgc ccgucacacc acgagaguuu guaacacccg aagucgguga 1440

gguaaccuuu uggagccagc cgccuaaggu gggacagaug auugggguga agucguaaca 1500

agguagccga uccggaaggu gcggcuggau caccuccuuu 1540

<210> 34
<211> 1332
<212> RNA
<213> Fusobacterium alocis

<220>
<221> modified_base
<222> (76)..(76)
<223> n is a, c, g, or u

<220>
<221> modified_base
<222> (80)..(80)
<223> n is a, c, g, or u

<220>
<221> modified_base
<222> (258)..(258)
<223> n is a, c, g, or u

<220>
<221> modified_base
<222> (261)..(261)
<223> n is a, c, g, or u

<220>
<221> modified_base
<222> (374)..(374)
<223> n is a, c, g, or u

<220>
<221> modified_base
<222> (812)..(812)
<223> n is a, c, g, or u

<220>
<221> modified_base
<222> (847)..(847)
<223> n is a, c, g, or u

<220>
<221> modified_base
<222> (999)..(999)
<223> n is a, c, g, or u

<220>
<221> modified_base
<222> (1076)..(1076)
<223> n is a, c, g, or u

<220>
<221> modified_base
<222> (1174)..(1174)
<223> n is a, c, g, or u

<400> 34
ggguggcgga cgggugagua acgcguaaag aacuugccuc acagauaggg acaacauuua 60

gaaaugaaug cuaaunccun auauuaugaa aauagggcau ccuauaauua ugaaagcuaa 120

augcgcugug agagagcuuu gcgucccauu agcuaguugg agagguaacg gcucaccaag 180

gcgaugaugg guagccggcc ugagagggug aacggccaca aggggacuga gacacggccc 240

uuacuccuac gggaggcngc nguggggaau auuggacaau ggaccgagag ucugauccag 300

caauucugug ugcacgauga aguuuuucgg aauguaaagu gcuuucaguu gggaagaaaa 360

gaaugacggu accnacagaa gaagugacgg cuaaauacgu gccagcagcc gcgguaauac 420

guaugucacg agcguuaucc ggauuuauug ggcguaaagc gcgucuaggu gguuauguaa 480

gucugaugug aaaaugcagg gcucaacucu guauugcguu ggaaacugug uaacuagagu 540

acuggagagg uaagcggaac uacaagugua gaggugaaau ucguagauau uuguaggaau 600

gccgaugggg aagccagcuu acuggacaga uacugacgcu aaagcgcgaa agcgugggua 660

gcaaacagga uuagauaccc ugguagucca cgccguaaac gaugauuacu agguguuggg 720

ggucgaaccu cagcgcccaa gcuaacgcga uaaguaaucc gccuggggag uacguacgca 780

aguaugaaac ucaaaggaau ugacggggac cngcacaagc gguggagcau gugguuuaau 840

ucgacgnaac gcgaggaacc uuaccagcgu uugacaucuu aggaaugaga uagagauaua 900

ucagugucuu cggggaaacc uaaagacagg uggugcaugg cugucgucag cucgugucgu 960

gagauguugg guuaaguccc gcaacgagcg caaccccunu cguauguuac caucauuaag 1020

uuggggacuc augcgauacu gccugcgaug agcaggagga agguggggau gacgunaagu 1080

caucaugccc cuuauacgcu gggcuacaca cgugcuacaa uggguagaac agagaguugc 1140

aaagccguga gguggagcua aucucagaaa acunuucuua guucggauug uacucugcaa 1200

cucgaguaca ugaaguugga aucgcuagua aucgcgaauc agcaaugucg cggugaauac 1260

guucucgggu cuuguacaca ccgcccguca caccacgaga guugguugca ccugaaguag 1320

caggccuaac cg 1332

<210> 35
<211> 1461
<212> RNA
<213> Fusobacterium nucleatum

<220>
<221> modified_base
<222> (288)..(288)
<223> n is a, c, g, or u

<220>
<221> modified_base
<222> (637)..(637)
<223> n is a, c, g, or u

<220>
<221> modified_base
<222> (744)..(746)
<223> n is a, c, g, or u

<220>
<221> modified_base
<222> (941)..(941)
<223> n is a, c, g, or u

<400> 35
auugaacgaa gaguuugauc cuggcucagg augaacgcug acagaaugcu uaacacaugc 60

aagucuacuu gaauuugggu uuuuuaacuu cgauuugggu ggcggacggg ugaguaacgc 120

guaaagaacu ugccucacag cuagggacaa cauuuggaaa cgaaugcuaa uaccuaauau 180

uaugauuaua gggcauccua gaauuaugaa agcuauaugc gcugugagag agcuuugcgu 240

cccauuagcu aguuggagag guaacggcuc accaaggcaa ugaugggnag ccggccugag 300

agggugaacg gccacaaggg gacugagaca cggcccuuac uccuacggga ggcagcagug 360

gggaauauug gacaauggac cgagagucug auccagcaau ucugugugca cgaugacguu 420

uuucggaaug uaaagugcuu ucaguuggga agaaaaaaau gacgguacca acagaagaag 480

ugacggcuaa auacgugcca gcagccgcgg uaauacguau gucacgagcg uuauccggau 540

uuauugggcg uaaagcgcgu cuaggugguu auguaagucu gaugugaaaa ugcagggcuc 600

aacucuguau ugcguuggaa acuguguaac uagaguncug gaaagguaag cggaacuaca 660

aguguagagg ugaaauucgu agauauuugu aggaaugccg auggggaagc cagcuuacug 720

gacagauacu gacgcugaag cgcnnnagcg uggguagcaa acaggauuag auacccuggu 780

aguccacgcc guaaacgaug auuacuaggu guuggggguc gaaccucagc gcccaagcaa 840

acgcgauaag uaauccgccu ggggaguacg uacgcaagua ugaaacucaa aggaauugac 900

ggggacccgc acaagcggug gagcaugugg uuuaauucga ngcaacgcga ggaaccuuac 960

cagcguuuga caucuuagga augagacaga gauguuucag ugucccuucg gggaaaccua 1020

aagacaggug gugcauggcu gucgucagcu cgugucguga gauguugggu uaagucccgc 1080

aacgagcgca accccuuucg uauguuacca ucauuaaguu ggggacucau gcgauacugc 1140

cuacgaugag uaggaggaag guggggauga cgucaaguca ucaugccccu uauacgcugg 1200

gcuacacacg ugcuacaaug gguagaacag agaguugcaa agccgugagg uggagcuaau 1260

cucagaaaac uauucuuagu ucggauugua cucugcaacu cgaguacaug aaguuggaau 1320

cgcuaguaau cgcgaaucag caaugucgcg gugaauacgu ucucgggucu uguacacacc 1380

gcccgucaca ccacgagagu ugguugcacc ugaaguagca ggccuaaccg uaaggaggga 1440

uguuccgagg gugugauuag c 1461

<210> 36
<211> 1452
<212> RNA
<213> Fusobacterium varium


<220>
<221> modified_base
<222> (256)..(256)
<223> n is a, c, g, or u

<220>
<221> modified_base
<222> (271)..(271)
<223> n is a, c, g, or u

<220>
<221> modified_base
<222> (320)..(320)
<223> n is a, c, g, or u

<220>
<221> modified_base
<222> (422)..(422)
<223> n is a, c, g, or u

<220>
<221> modified_base
<222> (509)..(509)
<223> n is a, c, g, or u

<220>
<221> modified_base
<222> (683)..(683)
<223> n is a, c, g, or u

<220>

<221> modified_base
<222> (844)..(844)
<223> n is a, c, g, or u

<220>
<221> modified_base
<222> (891)..(891)
<223> n is a, c, g, or u

<220>
<221> modified_base
<222> (923)..(926)
<223> n is a, c, g, or u

<220>
<221> modified_base
<222> (998)..(998)
<223> n is a, c, g, or u

<220>
<221> modified_base
<222> (1157)..(1158)
<223> n is a, c, g, or u

<220>
<221> modified_base
<222> (1447)..(1447)
<223> n is a, c, g, or u

<400> 36
aacgaagagu uugauccugg cucaggauga acgcugacag aaugcuuaac acaugcaagu 60

cuacuugauc uucgggugaa gguggcggac gggugaguaa cgcguaaaga acuugccuua 120

cagacuggga caacauuugg aaacgaaugc uaauaccgga uauuaugacu gagucgcaug 180

auuugguuau gaaagcuaua ugcgcuguga gagagcuuug cgucccauua guuaguuggu 240

gagguaacgg cucacnaaga cgaugauggg nagccggccu gagaggguga acggccacaa 300

ggggacugag acacggcccn yacuccuacg ggaggcagca guggggaaua uuggacaaug 360

gaccaaaagu cugauccagc aauucugugu gcacgaugaa guuuuucgga auguaaagug 420

cnuucaguug ggaagaaguc agugacggua ccaacagaag aagcgacggc uaaauacgug 480

ccagcagccg cgguaauacg uaugucgcna gcguuauccg gauuuauugg gcguaaagcg 540

cgucuaggcg guuuaguaag ucugauguga aaaugcgggg cucaaccccg uauugcguug 600

gaaacugcua aacuagagua cuggagaggu aggcggaacu acaaguguag aggugaaauu 660

cguagauauu uguaggaaug ccnaugggga agccagccua cuggacagau acugacgcua 720

aagcgcgaaa gcguggguag caaacaggau uagauacccu gguaguccac gccguaaacg 780

augauuacua gguguugggg gucgaaccuc agcgcccaag cuaacgcgau aaguaauccg 840

ccungggagu acguacgcaa guaugaaacu caaaggaauu gacggggacc ngcacaagcg 900

guggagcaug ugguuuaauu cgnnnnaacg cgaggaaccu uaccagcguu ugacauccca 960

agaaguuaac agagauguuu ucgugccucu ucggaggnac uuggugacag guggugcaug 1020

gcugucguca gcucgugucg ugagauguug gguuaagucc cgcaacgagc gcaaccccuu 1080

ucguauguua ccaucauuaa guuggggacu caugcgagac ugccugcgau gagcaggagg 1140

aaggugggga ugacgunnag ucaucaugcc ccuuauacgc ugggcuacac acgugcuaca 1200

auggguagua cagagagcug caaaccugcg aggguaagcu aaucucauaa aacuauucuu 1260

aguucggauu guacucugca acucgaguac augaaguugg aaucgcuagu aaucgcaaau 1320

cagcuauguu gcggugaaua cguucucggg ucuuguacac accgcccguc acaccacgag 1380

aguugguugc accugaagua acaggccuaa ccguaaggag ggauguuccg agggugugau 1440

uagcganugg gg 1452

<210> 37
<211> 20
<212> DNA
<213> Artificial Sequence

<220>
<223> Description of Artificial Sequence: Synthetic
      oligonucleotide

<400> 37
agagtttgat cctggctcag 20

<210> 38
<211> 18
<212> DNA
<213> Artificial Sequence

<220>
<223> Description of Artificial Sequence: Synthetic
      oligonucleotide

<400> 38
gctgcctccc gtaggagt 18